# EUROPEAN PATENT APPLICATION

(11) **EP 2 402 367 A1**
(43) Date of publication of application: **04.01.2012**
(21) Application number: 11168475.9
(22) Date of filing: 26.09.2006
(51) Int. Cl.: C07K 14/245, C12Q 1/18, C40B 30/04, C40B 40/10, G01N 33/566, G01N 33/58

(54) **Library from toxin mutants, and methods of using same**

(30) Priority: 26.09.2005 US 720924 P
(62) Divisional of application: 06790746.9
(71) Applicant: Molecular Templates, Inc., Georgetown, TX 78626 (US)
(72) Inventor: Gariepy, Jean, Toronto, Ontario M5P 2L5 (CA); Revers, Leigh, Toronto, Ontario M4Y 3C4 (CA)
(74) Representative: Modiano, Micaela Nadia

(57) **Abstract**

This application relates to libraries of ABx toxin mutants, in which a peptide insert is introduced into the protease- sensitive loop of the A-chain sequence to alter the type of cells to which toxic species are delivered. Said libraries are used in the development of therapeutics targeted against specific cell types.

SEQUENCE LISTING

<110>  UNIVERSITY HEALTH NETWORK

<120>  Library From Toxin Mutants, and Methods of Using Same

<130>  34104-0094

<140>  PCT/CA2006/001581
<141>  2006-09-26

<160>  88

<170>  PatentIn version 3.4

<210>  1
<211>  293
<212>  PRT
<213>  Artificial

<220>
<223>  Shiga-like toxin

<400>  1

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Ala Ser Arg Val Ala Arg Met Ala Ser Asp Glu
                245                 250                 255

Phe Pro Ser Met Cys Pro Ala Asp Gly Arg Val Arg Gly Ile Thr His
            260                 265                 270

Asn Lys Ile Leu Trp Asp Ser Ser Thr Leu Gly Ala Ile Leu Met Arg
        275                 280                 285

Arg Thr Ile Ser Ser
    290

<210>  2
<211>  32
<212>  DNA
<213>  Artificial

<220>
<223>  Primer A

<400>  2
gttactgtga cagctgaagc tttacgtttt cg                                32

<210>  3
<211>  31
<212>  DNA
<213>  Artificial

<220>
<223>  Primer B

<400>  3
gagaagaaga gactgcagat tccatctgtt g                                 31

<210>  4
<211>  0
<212>  DNA
<213>  Artificial

<220>
<223>  SAM3

<400>  4
000

<210>  5
<211>  0
<212>  PRT
<213>  Artificial

<220>
<223>  SAM5

<400>  5
000
<210>  6
<211>  7
<212>  PRT
<213>  Artificial

<220>
<223>  Mutant Shiga-like toxin protein

<400>  6

Ile Tyr Ser Asn Lys Leu Met
1               5


<210>  7
<211>  7
<212>  PRT
<213>  Artificial

<220>
<223>  Mutant Shiga-like toxin protein

<400>  7

Ala Ala Phe Ala Asp Leu Ile
1               5


<210>  8
<211>  300
<212>  PRT
<213>  Artificial

<220>
<223>  Shiga-like toxin 1 A with the breast cancer-associated MUC1
       epitope PDTRPAP less the N-terminus tags

<400>  8

Lys Glu Phe Thr Leu Asp Phe Ser Thr Ala Lys Thr Tyr Val Asp Ser
1               5                   10                  15


Leu Asn Val Ile Arg Ser Ala Ile Gly Thr Pro Leu Gln Thr Ile Ser
            20                  25                  30


Ser Gly Gly Thr Ser Leu Leu Met Ile Asp Ser Gly Ser Gly Asp Asn
        35                  40                  45


Leu Phe Ala Val Asp Val Arg Gly Ile Asp Pro Glu Glu Gly Arg Phe
    50                  55                  60


Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Pro Asp Thr Arg Pro Ala Pro Ala Ser Arg Val
                245                 250                 255

Ala Arg Met Ala Ser Asp Glu Phe Pro Ser Met Cys Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  9
<211>  319
<212>  PRT
<213>  Artificial

<220>
<223>  Shiga-like toxin with the breast cancer-associated MUC1 epitope
       PDTRPAPwith an example of one possible N-terminus tag

<400>  9

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Thr Arg Pro Ala Pro Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  10
<211>  319
<212>  PRT
<213>  Artificial

<220>
<223>  Shiga-like toxin 1 A chain with the breast cancer-associated MUC1
       epitope PDTRPAP with an example of one possible N-terminus

<400>  10

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln

180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Thr Arg Pro Ala Pro Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  11
<211>  300
<212>  PRT
<213>  Artificial

<220>
<223>  SLT-1 A chain-tripeptide library construction where the three
       positions of the MUC1 epitope recognized by the mAb Onc M27 are
       randomized less the N-terminus

<220>
<221>  misc_feature
<222>  (248)..(250)
<223>  X is any amino acid

<400>  11

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Pro Asp Xaa Xaa Xaa Ala Pro Ala Ser Arg Val
                245                 250                 255

Ala Arg Met Ala Ser Asp Glu Phe Pro Ser Met Cys Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  12
<211>  319
<212>  PRT
<213>  Artificial

<220>
<223>  SLT-1A chain-tripeptide library construction where the three

positions of the MUC1 epitope recognized by the mAb Onc M27 are
randomized with an example of one possible N-terminus tag

<220>
<221>  misc_feature
<222>  (267)..(269)
<223>  X is any amino acid

<400>  12

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val

225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Xaa Xaa Xaa Ala Pro Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  13
<211>  319
<212>  PRT
<213>  Artificial

<220>
<223>  SLT-A chain tripeptide library construction where the three
       psotions of the MUC 1 epitope recognized by the mAb Onc M27 are
       randomized with an example of one possible N-terminus tag

<220>
<221>  misc_feature
<222>  (267)..(269)
<223>  X is any amino acid

<400>  13

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Xaa Xaa Xaa Ala Pro Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  14
<211>  300
<212>  PRT
<213>  Artificial

<220>
<223>  SLT-1A  chain tripeptide library wherein cysteine 261 has been
       mutated to an alanine and where the C242 residue has been
       biotinylated less the N-terminus tag

<220>
<221>  misc_feature
<222>  (248)..(250)
<223>  X is any amino acid

<400>  14

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Pro Asp Xaa Xaa Xaa Ala Pro Ala Ser Arg Val
                245                 250                 255

Ala Arg Met Ala Ser Asp Glu Phe Pro Ser Met Ala Pro Ala Asp Gly

260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  15
<211>  319
<212>  PRT
<213>  Artificial

<220>
<223>  SLT-1A chain tripeptide library wherein cysteine 261 has been
       mutated to an alanine and where the C242 residue has been
       biotinylated with an example of one possible N-terminus tag

<220>
<221>  misc_feature
<222>  (267)..(269)
<223>  X is any amino acid

<400>  15

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Asn Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Xaa Xaa Xaa Ala Pro Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315


<210>  16
<211>  319
<212>  PRT
<213>  Artificial

<220>
<223>  SLT-1A chain tripeptide library wherein cysteine 261 has been
       mutated to an alanine and where the C242 residue has been
       biotinylated with an example of one possible N-terminus tag


<220>
<221>  misc_feature
<222>  (267)..(269)
<223>  X is any amino acid

<400>  16

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln

35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Asn Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Xaa Xaa Xaa Ala Pro Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser

305                 310                 315


<210>  17
<211>  300
<212>  PRT
<213>  Artificial

<220>
<223>  SLT - 1A chain tripeptide library construction wherein cysteine
       242 has been mutated to an alanine less an N-terminus tags


<220>
<221>  misc_feature
<222>  (248)..(250)
<223>  X is any amino acid

<400>  17

Lys Glu Phe Thr Leu Asp Phe Ser Thr Ala Lys Thr Tyr Val Asp Ser
1               5                   10                  15


Leu Asn Val Ile Arg Ser Ala Ile Gly Thr Pro Leu Gln Thr Ile Ser
            20                  25                  30


Ser Gly Gly Thr Ser Leu Leu Met Ile Asp Ser Gly Ser Gly Asp Asn
        35                  40                  45


Leu Phe Ala Val Asp Val Arg Gly Ile Asp Pro Glu Glu Gly Arg Phe
    50                  55                  60


Asn Asn Leu Arg Leu Ile Val Glu Arg Asn Asn Leu Tyr Val Thr Gly
65                  70                  75                  80


Phe Val Asn Arg Thr Asn Asn Val Phe Tyr Arg Phe Ala Asp Phe Ser
                85                  90                  95


His Val Thr Phe Pro Gly Thr Thr Ala Val Thr Leu Ser Gly Asp Ser
            100                 105                 110


Ser Tyr Thr Thr Leu Gln Arg Val Ala Gly Ile Ser Arg Thr Gly Met
        115                 120                 125


Gln Ile Asn Arg His Ser Leu Thr Thr Ser Tyr Leu Asp Leu Met Ser
    130                 135                 140


His Ser Gly Thr Ser Leu Thr Gln Ser Val Ala Arg Ala Met Leu Arg
145                 150                 155                 160


Phe Val Thr Val Thr Ala Glu Ala Leu Arg Phe Arg Gln Ile Gln Arg
                165                 170                 175


Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Pro Asp Xaa Xaa Xaa Ala Pro Ala Ser Arg Val
                245                 250                 255

Ala Arg Met Ala Ser Asp Glu Phe Pro Ser Met Cys Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  18
<211>  319
<212>  PRT
<213>  Artificial

<220>
<223>  SLT -1 A chain tripeptide library construction wherein cysteine
       242 has been mutated to an alanine with an example of one
       possible N-terminus tag

<220>
<221>  misc_feature
<222>  (267)..(269)
<223>  X is any amino acid

<400>  18

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Pro Asp Xaa Xaa Xaa Ala Pro Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  19
<211>  319
<212>  PRT
<213>  Artificial

<220>
<223>  SLT -1 A chain tripeptide library construction wherein cysteine
       242 has been mutated to an alanine with an example of one
       possible N-terminus tag

<220>
<221>  misc_feature
<222>  (267)..(269)
<223>  X is any amino acid

<400>  19

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Pro Asp Xaa Xaa Xaa Ala Pro Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  20
<211>  300
<212>  PRT
<213>  Artificial

<220>
<223>  SLT- 1 A chain tripeptide library construction wherein cysteine
       242 and cysteine 261 have both been mutated to alanine residues
       less the N-terminus tag

<220>
<221>  misc_feature
<222>  (248)..(250)
<223>  X is any amino acid

<400>  20

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Pro Asp Xaa Xaa Xaa Ala Pro Ala Ser Arg Val
                245                 250                 255

Ala Arg Met Ala Ser Asp Glu Phe Pro Ser Met Ala Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  21
<211>  319
<212>  PRT
<213>  Artificial

<220>
<223>  SLT- 1 A chain tripeptide library construction wherein cysteine
       242 and cysteine 261 have both been mutated to alanine residues
       with an exampel of one possible N-terminus tag

<220>
<221>  misc_feature
<222>  (267)..(269)
<223>  X is any amino acid

<400>  21

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Pro Asp Xaa Xaa Xaa Ala Pro Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  22
<211>  319
<212>  PRT
<213>  Artificial

<220>
<223>  SLT- 1 A chain tripeptide library construction wherein cysteine
       242 and cysteine 261 have both been mutated to alanine residues
       with an example of one possible N-terminus tag

<220>
<221>  misc_feature
<222>  (267)..(269)
<223>  X is any amino acid

<400>  22

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
            245                 250                 255

Leu Ile Leu Asn Ala His His His Pro Asp Xaa Xaa Xaa Ala Pro Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  23
<211>  258
<212>  PRT
<213>  Artificial

<220>
<223>  SLT-1 A1 chain with the breast cancer-associated MUC1 epitope
       PDTRPAP inserted between residues 245 and 246 and a Histidine tag
       followed by a protease cleavage site less a N-terminus tags

<400>  23

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Pro Asp Thr Arg Pro Ala Pro Ala Ser Arg Val
                245                 250                 255

Ala Arg

<210>  24
<211>  277
<212>  PRT
<213>  Artificial

<220>
<223>  SLT-1 A1 chain with the breast cancer-associated MUC1 epitope
       PDTRPAP inserted between residues 245 and 246 and a Histidine tag
       followed by a protease cleavage site with an example of one
       possible N-terminus tag

<400>  24

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr

            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Thr Arg Pro Ala Pro Ala
            260                 265                 270

Ser Arg Val Ala Arg
        275

<210> 25

<211>  277
<212>  PRT
<213>  Artificial

<220>
<223>  SLT-1 A1 chain with the breast cancer-associated MUC1 epitope
       PDTRPAP inserted between residues 245 and 246 and a Histidine tag
       followed by a protease cleavage site with an example of one
       N-terminus tag

<400>  25

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Thr Arg Pro Ala Pro Ala
            260                 265                 270

Ser Arg Val Ala Arg
        275

<210>  26
<211>  258
<212>  PRT
<213>  Artificial

<220>
<223>  SLT-1 A1 chain tripeptide library construction where the three
       key positions of the MUC1 epitope recognized by the mAb Onc M27
       were randomized and wherein cysteine 242 has been biotinylated
       less N-terminus tags

<220>
<221>  misc_feature
<222>  (248)..(250)
<223>  X is any amino acid

<400>  26

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser

130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Asn
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Pro Asp Xaa Xaa Xaa Ala Pro Ala Ser Arg Val
                245                 250                 255

Ala Arg

<210>  27
<211>  277
<212>  PRT
<213>  Artificial

<220>
<223>  SLT-1 A1 chain tripeptide library construction where the three
       key positions of the MUC1 epitope recognized by the mAb Onc M27
       were randomized and wherein cysteine 242 has been biotinylated
       with an example of one possible N-terminus tag

<220>
<221>  misc_feature
<222>  (267)..(269)
<223>  X is any amino acid

<400>  27

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Leu Ser Ser Tyr Thr Thr Leu Gln Arg Val Ala Gly Ile Ser Arg
    130                 135                 140

Thr Gly Asn Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Xaa Xaa Xaa Ala Pro Ala
            260                 265                 270

Ser Arg Val Ala Arg
        275

<210>  28
<211>  277
<212>  PRT
<213>  Artificial

<220>
<223>  SLT-1 A1 chain tripeptide library construction where the three
       key positions of the MUC1 epitope recognized by the mAb Onc M27
       were randomized and wherein cysteine 242 has been biotinylated
       with an example of one possible N-terminus tag

<220>
<221>  misc_feature
<222>  (267)..(269)
<223>  X is any amino avid

<400>  28

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Leu Ser Ser Tyr Thr Thr Leu Gln Arg Val Ala Gly Ile Ser Arg
    130                 135                 140

Thr Gly Asn Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Pro Asp Xaa Xaa Xaa Ala Pro Ala
            260                 265                 270

Ser Arg Val Ala Arg
        275

<210>  29
<211>  258
<212>  PRT
<213>  Artificial

<220>
<223>  SLT-1 A1 chain tripeptide library construction wherein cysteine
       242 has been mutated to an alanine less the N-terminus tag

<220>
<221>  misc_feature
<222>  (248)..(251)
<223>  X is any amino acid

<400>  29

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Asn
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Pro Asp Xaa Xaa Xaa Ala Pro Ala Ser Arg Val
                245                 250                 255

Ala Arg

<210>  30
<211>  277
<212>  PRT
<213>  Artificial

<220>
<223>  SLT-1 A1 chain tripeptide library construction wherein cysteine
       242 has been mutated to an alanine with an example of one
       N-terminus tag

<220>
<221>  misc_feature
<222>  (267)..(269)
<223>  X is any amino acid

<400>  30

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Pro Asp Xaa Xaa Xaa Ala Pro Ala
            260                 265                 270

Ser Arg Val Ala Arg
        275

<210>  31
<211>  277
<212>  PRT
<213>  Artificial

<220>
<223>  SLT-1 A1 chain tripeptide library construction wherein cysteine
       242 has been mutated to an alanine with an example of one
       N-terminus tag

<220>
<221>  misc_feature
<222>  (267)..(269)
<223>  X is any amino acid

<400>  31

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Pro Asp Xaa Xaa Xaa Ala Pro Ala
            260                 265                 270

Ser Arg Val Ala Arg
        275

<210>  32
<211>  300
<212>  PRT
<213>  Artificial

<220>
<223>  7 amino acid random segment inserted between residues 245 and 246
       of the SLT-1 A chain less the N-terminus tag

<220>
<221>  misc_feature
<222>  (246)..(252)
<223>  X is any amino acid

<400>  32

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met

180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Xaa Xaa Xaa Xaa Xaa Xaa Xaa Ala Ser Arg Val
                245                 250                 255

Ala Arg Met Ala Ser Asp Glu Phe Pro Ser Met Cys Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  33
<211>  319
<212>  PRT
<213>  Artificial

<220>
<223>  7 amino acid random segment inserted between residues 245 and 246
       of the SLT-1 A chain with an example of one possible N-terminus
       tag

<220>
<221>  misc_feature
<222>  (265)..(271)
<223>  X is any amino acid

<400>  33

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Xaa Xaa Xaa Xaa Xaa Xaa Xaa Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  34
<211>  319
<212>  PRT
<213>  Artificial

<220>
<223>  7 amino acid random segment inserted between residues 245 and 246

of the SLT-1 A chain with an example of an N-terminus tag

<220>
<221>  misc_feature
<222>  (265)..(271)
<223>  X is any amino acid

<400>  34

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Xaa Xaa Xaa Xaa Xaa Xaa Xaa Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315


<210>  35
<211>  300
<212>  PRT
<213>  Artificial

<220>
<223>  7 amino acid random segment inserted between residues 245 and 246
       of the SLT-1 A chain, wherein the cysteine at position 261 has
       been mutated to an alanine and the cysteine at position 242 has
       been biotinylated less an N-terminus tag


<220>
<221>  misc_feature
<222>  (246)..(252)
<223>  X is any amino acid

<400>  35

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Xaa Xaa Xaa Xaa Xaa Xaa Xaa Ala Ser Arg Val
                245                 250                 255

Ala Arg Met Ala Ser Asp Glu Phe Pro Ser Met Ala Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  36
<211>  319
<212>  PRT
<213>  Artificial

<220>
<223>  7 amino acid random segment inserted between residues 245 and 246
       of the SLT-1 A chain, wherein the cysteine at position 261 has
       been mutated to an alanine and the cysteine at position 242 has
       been biotinylated with an example of one N-terminus tag

<220>
<221>  misc_feature
<222>  (265)..(265)
<223>  Xaa can be any naturally occurring amino acid

<220>
<221>  misc_feature

<222>  (266)..(272)
<223>  X is any amino acid

<400>  36

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Xaa Xaa Xaa Xaa Xaa Xaa Xaa Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  37
<211>  319
<212>  PRT
<213>  Artificial

<220>
<223>  7 amino acid random segment inserted between residues 245 and 246
       of the SLT-1 A chain, wherein the cysteine at position 261 has
       been mutated to an alanine and the cysteine at position 242 has
       been biotinylated with an example of one N-terminus tag

<220>
<221>  misc_feature
<222>  (265)..(265)
<223>  Xaa can be any naturally occurring amino acid

<220>
<221>  misc_feature
<222>  (266)..(272)
<223>  X is any amino acid

<400>  37

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Xaa Xaa Xaa Xaa Xaa Xaa Xaa Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  38
<211>  300
<212>  PRT
<213>  Artificial

<220>
<223>  7 amino acid random segment inserted between residues 245 and 246
       of the SLT-1 A chain, wherein the cysteine at position 242 has
       been mutated to an alanine less the N-terminus tag

<220>
<221>  misc_feature
<222>  (246)..(252)
<223>  X is any amino acid

<400> 38

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Xaa Xaa Xaa Xaa Xaa Xaa Xaa Ala Ser Arg Val
                245                 250                 255

Ala Arg Met Ala Ser Asp Glu Phe Pro Ser Met Cys Pro Ala Asp Gly

260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  39
<211>  319
<212>  PRT
<213>  Artificial

<220>
<223>  7 amino acid random segment inserted between residues 245 and 246
       of the SLT-1 A chain, wherein the cysteine at position 242 has
       been mutated to an alanine with an example of one N-terminus tag

<220>
<221>  misc_feature
<222>  (265)..(271)
<223>  X is any amino acid

<400>  39

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Xaa Xaa Xaa Xaa Xaa Xaa Xaa Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  40
<211>  319
<212>  PRT
<213>  Artificial

<220>
<223>  7 amino acid random segment inserted between residues 245 and 246
       of the SLT-1 A chain, wherein the cysteine at position 242 has
       been mutated to an alanine with an example of one N-terminus tag

<220>
<221>  misc_feature
<222>  (265)..(271)
<223>  X is any amino acid

<400>  40

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln

35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Xaa Xaa Xaa Xaa Xaa Xaa Xaa Ala
            260                 265                 270

Ser Arg Val Ala Arg Met Ala Ser Asp Glu Phe Pro Ser Met Cys Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser

305                 310                 315


<210>  41
<211>  300
<212>  PRT
<213>  Artificial

<220>
<223>  7 amino acid random segment inserted between 245 and 246 of the
       SLT -1 A chain, wherein the cysteine at positions 242 and 261
       have been mutated to alanines less the N-terminus tags


<220>
<221>  misc_feature
<222>  (246)..(252)
<223>  X is any amino acid

<400>  41

Lys Glu Phe Thr Leu Asp Phe Ser Thr Ala Lys Thr Tyr Val Asp Ser
1               5                   10                  15


Leu Asn Val Ile Arg Ser Ala Ile Gly Thr Pro Leu Gln Thr Ile Ser
            20                  25                  30


Ser Gly Gly Thr Ser Leu Leu Met Ile Asp Ser Gly Ser Gly Asp Asn
        35                  40                  45


Leu Phe Ala Val Asp Val Arg Gly Ile Asp Pro Glu Glu Gly Arg Phe
    50                  55                  60


Asn Asn Leu Arg Leu Ile Val Glu Arg Asn Asn Leu Tyr Val Thr Gly
65                  70                  75                  80


Phe Val Asn Arg Thr Asn Asn Val Phe Tyr Arg Phe Ala Asp Phe Ser
                85                  90                  95


His Val Thr Phe Pro Gly Thr Thr Ala Val Thr Leu Ser Gly Asp Ser
            100                 105                 110


Ser Tyr Thr Thr Leu Gln Arg Val Ala Gly Ile Ser Arg Thr Gly Met
        115                 120                 125


Gln Ile Asn Arg His Ser Leu Thr Thr Ser Tyr Leu Asp Leu Met Ser
    130                 135                 140


His Ser Gly Thr Ser Leu Thr Gln Ser Val Ala Arg Ala Met Leu Arg
145                 150                 155                 160


Phe Val Thr Val Thr Ala Glu Ala Leu Arg Phe Arg Gln Ile Gln Arg
                165                 170                 175


Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Xaa Xaa Xaa Xaa Xaa Xaa Xaa Ala Ser Arg Val
                245                 250                 255

Ala Arg Met Ala Ser Asp Glu Phe Pro Ser Met Ala Pro Ala Asp Gly
            260                 265                 270

Arg Val Arg Gly Ile Thr His Asn Lys Ile Leu Trp Asp Ser Ser Thr
        275                 280                 285

Leu Gly Ala Ile Leu Met Arg Arg Thr Ile Ser Ser
    290                 295                 300

<210>  42
<211>  319
<212>  PRT
<213>  Artificial

<220>
<223>  7 amino acid random segment inserted between 245 and 246 of the
       SLT -1 A chain, wherein the cysteine at positions 242 and 261
       have been mutated to alanines with an example of one N-terminus
       tag

<220>
<221>  misc_feature
<222>  (265)..(271)
<223>  X is any amino acid

<400>  42

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Xaa Xaa Xaa Xaa Xaa Xaa Xaa Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  43
<211>  319
<212>  PRT
<213>  Artificial

<220>
<223>  7 amino acid random segment inserted between 245 and 246 of the
       SLT -1 A chain, wherein the cysteine at positions 242 and 261

have been mutated to alanines with an example of one N-terminus
tag

<220>
<221>  misc_feature
<222>  (265)..(271)
<223>  X is any amino acid

<400>  43

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val

225                 230                 235                 240

Gly Arg Ile Ser Phe Gly Ser Ile Asn Ala Ile Leu Gly Ser Val Ala
                245                 250                 255

Leu Ile Leu Asn Ala His His His Xaa Xaa Xaa Xaa Xaa Xaa Xaa Ala
            260                 265                 270

Ser Arg Val Ala Arg Met Ala Ser Asp Glu Phe Pro Ser Met Ala Pro
        275                 280                 285

Ala Asp Gly Arg Val Arg Gly Ile Thr His Asn Lys Ile Leu Trp Asp
    290                 295                 300

Ser Ser Thr Leu Gly Ala Ile Leu Met Arg Arg Thr Ile Ser Ser
305                 310                 315

<210>  44
<211>  258
<212>  PRT
<213>  Artificial

<220>
<223>  7 amino acid random segment inserted between residues 245 and 246
       of the SLT-1 A1 chain, wherein the cysteine residue at position
       242 has been biotinylated less the N-terminus tags

<220>
<221>  misc_feature
<222>  (246)..(252)
<223>  X is any amino acid

<400>  44

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Xaa Xaa Xaa Xaa Xaa Xaa Xaa Ala Ser Arg Val
                245                 250                 255

Ala Arg

<210>  45
<211>  277
<212>  PRT
<213>  Artificial

<220>
<223>  7 amino acid random segment inserted between residues 245 and 246
       of the SLT-1 A1 chain, wherein the cysteine residue at position
       242 has been biotinylated with an example of one N-terminus tag

<220>
<221>  misc_feature
<222>  (265)..(271)
<223>  X is any amino acid

<400>  45

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln

35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Xaa Xaa Xaa Xaa Xaa Xaa Xaa Ala
            260                 265                 270

Ser Arg Val Ala Arg
        275

<210>  46
<211>  277
<212>  PRT
<213>  Artificial

<220>
<223>  7 amino acid random segment inserted between residues 245 and 246
       of the SLT-1 A1 chain, wherein the cysteine residue at position
       242 has been biotinylated with an example of one N-terminus tag

<220>
<221>  misc_feature
<222>  (265)..(271)
<223>  X is any amino acid

<400>  46

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Xaa Xaa Xaa Xaa Xaa Xaa Xaa Ala
            260                 265                 270

Ser Arg Val Ala Arg
        275

<210>  47
<211>  258
<212>  PRT
<213>  Artificial

<220>
<223>  7 amino acid random segment inserted between residues 245 and 246
       of the SLT-1 A1 chain, wherein the cysteine residue at position
       242 has been mutated to an alanine less the N-terminus tags

<220>
<221>  misc_feature
<222>  (246)..(252)
<223>  X is any amino acid

<400>  47

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser

130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Xaa Xaa Xaa Xaa Xaa Xaa Xaa Ala Ser Arg Val
                245                 250                 255

Ala Arg

<210>  48
<211>  277
<212>  PRT
<213>  Artificial

<220>
<223>  7 amino acid random segment inserted between residues 245 and 246
       of the SLT-1 A1 chain, wherein the cysteine residue at position
       242 has been mutated to an alanine with an example of one
       N-terminus tag

<220>
<221>  misc_feature
<222>  (265)..(271)
<223>  X is any amino acid

<400>  48

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Xaa Xaa Xaa Xaa Xaa Xaa Xaa Ala
            260                 265                 270

Ser Arg Val Ala Arg
        275

<210>  49
<211>  277
<212>  PRT
<213>  Artificial

<220>
<223>  7 amino acid random segment inserted between residues 245 and 246
       of the SLT-1 A1 chain, wherein the cysteine residue at position
       242 has been mutated to an alanine with an example of one
       N-terminus tag

<220>
<221>  misc_feature
<222>  (265)..(271)
<223>  X is any amino acid

<400>  49

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Xaa Xaa Xaa Xaa Xaa Xaa Xaa Ala
            260                 265                 270

Ser Arg Val Ala Arg
        275

<210>  50
<211>  293
<212>  PRT
<213>  Artificial

<220>
<223>  Amino acid sequence corresponding to the SLT-1 A region, with
       cysteine 261 mutated to an alanine

<400>  50

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Ala Ser Arg Val Ala Arg Met Ala Ser Asp Glu
                245                 250                 255

Phe Pro Ser Met Ala Pro Ala Asp Gly Arg Val Arg Gly Ile Thr His
            260                 265                 270

Asn Lys Ile Leu Trp Asp Ser Ser Thr Leu Gly Ala Ile Leu Met Arg
        275                 280                 285

Arg Thr Ile Ser Ser
    290

<210>  51
<211>  312
<212>  PRT
<213>  Artificial

<220>
<223>  Amino acid sequence corresponding to the SLT-1 A region, with
       cysteine 261 mutated to an alanine, and with an example of one
       N-terminus tag

<400>  51

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Ala Ser Arg Val Ala Arg Met Ala
            260                 265                 270

Ser Asp Glu Phe Pro Ser Met Ala Pro Ala Asp Gly Arg Val Arg Gly
        275                 280                 285

Ile Thr His Asn Lys Ile Leu Trp Asp Ser Ser Thr Leu Gly Ala Ile
    290                 295                 300

Leu Met Arg Arg Thr Ile Ser Ser
305                 310

<210>  52
<211>  312
<212>  PRT
<213>  Artificial

<220>
<223>  Amino acid sequence corresponding to the SLT-1 A region, with
       cysteine 261 mutated to an alanine, and with an example of one
       N-terminus tag

<400>  52
   Lys Gly Met Arg Ser His His His His His His His His Arg Val Ala
   1               5                   10                  15
   Arg Ala Ser Lys Glu Phe Thr Leu Asp Phe Ser Thr Ala Lys Thr Tyr
               20                  25                  30
   Val Asp Ser Leu Asn Val Ile Arg Ser Ala Ile Gly Thr Pro Leu Gln
           35                  40                  45
   Thr Ile Ser Ser Gly Gly Thr Ser Leu Leu Met Ile Asp Ser Gly Ser
       50                  55                  60
   Gly Asp Asn Leu Phe Ala Val Asp Val Arg Gly Ile Asp Pro Glu Glu
   65                  70                  75                  80
   Gly Arg Phe Asn Asn Leu Arg Leu Ile Val Glu Arg Asn Asn Leu Tyr
                   85                  90                  95
   Val Thr Gly Phe Val Asn Arg Thr Asn Asn Val Phe Tyr Arg Phe Ala
               100                 105                 110
   Asp Phe Ser His Val Thr Phe Pro Gly Thr Thr Ala Val Thr Leu Ser
           115                 120                 125
   Gly Asp Ser Ser Tyr Thr Thr Leu Gln Arg Val Ala Gly Ile Ser Arg
       130                 135                 140
   Thr Gly Met Gln Ile Asn Arg His Ser Leu Thr Thr Ser Tyr Leu Asp
   145                 150                 155                 160
   Leu Met Ser His Ser Gly Thr Ser Leu Thr Gln Ser Val Ala Arg Ala
                   165                 170                 175
   Met Leu Arg Phe Val Thr Val Thr Ala Glu Ala Leu Arg Phe Arg Gln
               180                 185                 190
   Ile Gln Arg Gly Phe Arg Thr Thr Leu Asp Asp Leu Ser Gly Arg Ser
           195                 200                 205
   Tyr Val Met Thr Ala Glu Asp Val Asp Leu Thr Leu Asn Trp Gly Arg
       210                 215                 220
   Leu Ser Ser Val Leu Pro Asp Tyr His Gly Gln Asp Ser Val Arg Val
   225                 230                 235                 240
   Gly Arg Ile Ser Phe Gly Ser Ile Asn Ala Ile Leu Gly Ser Val Ala
                   245                 250                 255
   Leu Ile Leu Asn Cys His His His Ala Ser Arg Val Ala Arg Met Ala
               260                 265                 270
   Ser Asp Glu Phe Pro Ser Met Ala Pro Ala Asp Gly Arg Val Arg Gly
           275                 280                 285
   Ile Thr His Asn Lys Ile Leu Trp Asp Ser Ser Thr Leu Gly Ala Ile
       290                 295                 300
   Leu Met Arg Arg Thr Ile Ser Ser
   305                 310

<210>  53
<211>  293
<212>  PRT
<213>  Artificial

<220>
<223>  Amino acid sequence corresponding to SLT-1 A region, with
       cysteine 242 mutated to alanine

<400>  53

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met

180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Ala Ser Arg Val Ala Arg Met Ala Ser Asp Glu
                245                 250                 255

Phe Pro Ser Met Cys Pro Ala Asp Gly Arg Val Arg Gly Ile Thr His
            260                 265                 270

Asn Lys Ile Leu Trp Asp Ser Ser Thr Leu Gly Ala Ile Leu Met Arg
        275                 280                 285

Arg Thr Ile Ser Ser
    290

<210>  54
<211>  312
<212>  PRT
<213>  Artificial

<220>
<223>  Amino acid sequence corresponding to SLT-1 A region, with
       cysteine 242 mutated to alanine with an example of one N-terminus
       tag

<400>  54

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala

            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Ala Ser Arg Val Ala Arg Met Ala
            260                 265                 270

Ser Asp Glu Phe Pro Ser Met Cys Pro Ala Asp Gly Arg Val Arg Gly
        275                 280                 285

Ile Thr His Asn Lys Ile Leu Trp Asp Ser Ser Thr Leu Gly Ala Ile
    290                 295                 300

Leu Met Arg Arg Thr Ile Ser Ser
305                 310

<210> 55
<211> 312
<212> PRT
<213> Artificial

<220>
<223> Amino acid sequence corresponding to SLT-1 A region, with
cysteine 242 mutated to alanine with an example of one N-terminus
tag

<400> 55

Lys Gly Met Arg Ser His His His His His His His His Arg Val Ala

1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Ala Ser Arg Val Ala Arg Met Ala
            260                 265                 270

Ser Asp Glu Phe Pro Ser Met Cys Pro Ala Asp Gly Arg Val Arg Gly

275                 280                 285

Ile Thr His Asn Lys Ile Leu Trp Asp Ser Ser Thr Leu Gly Ala Ile
    290                 295                 300

Leu Met Arg Arg Thr Ile Ser Ser
305                 310

<210>  56
<211>  293
<212>  PRT
<213>  Artificial

<220>
<223>  Amino acid sequence corresponding to the SLT-1 A region, with
       cysteines 242 and 261 mutated to alanines

<400>  56

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Ala Ser Arg Val Ala Arg Met Ala Ser Asp Glu
                245                 250                 255

Phe Pro Ser Met Cys Pro Ala Asp Gly Arg Val Arg Gly Ile Thr His
            260                 265                 270

Asn Lys Ile Leu Trp Asp Ser Ser Thr Leu Gly Ala Ile Leu Met Arg
        275                 280                 285

Arg Thr Ile Ser Ser
    290

<210>  57
<211>  312
<212>  PRT
<213>  Artificial

<220>
<223>  Amino acid sequence corresponding to the SLT-1 A region, with
       cysteines 242 and 261 mutated to alanines with an example of one
       possible N-terminus tag

<400>  57

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Ala Ser Arg Val Ala Arg Met Ala
            260                 265                 270

Ser Asp Glu Phe Pro Ser Met Cys Pro Ala Asp Gly Arg Val Arg Gly
        275                 280                 285

Ile Thr His Asn Lys Ile Leu Trp Asp Ser Ser Thr Leu Gly Ala Ile
    290                 295                 300

Leu Met Arg Arg Thr Ile Ser Ser
305                 310

<210>  58
<211>  312
<212>  PRT
<213>  Artificial

<220>
<223>  Amino acid sequence corresponding to the SLT-1 A region, with
       cysteines 242 and 261 mutated to alanines with an example of one
       N-terminus tag

<400>  58

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Ala Ser Arg Val Ala Arg Met Ala
            260                 265                 270

Ser Asp Glu Phe Pro Ser Met Cys Pro Ala Asp Gly Arg Val Arg Gly
        275                 280                 285

Ile Thr His Asn Lys Ile Leu Trp Asp Ser Ser Thr Leu Gly Ala Ile
    290                 295                 300

Leu Met Arg Arg Thr Ile Ser Ser
305                 310

<210>  59
<211>  251
<212>  PRT
<213>  Artificial

<220>
<223>  Amino acid sequence corresponding to the SLT-1 A1 region

<400>  59

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Cys His His His Ala Ser Arg Val Ala Arg
                245                 250

<210>  60
<211>  270
<212>  PRT
<213>  Artificial

<220>
<223>  Amino acid sequence corresponding to the SLT-1 A1 region with an
       example of one possible N-terminus tag

<400>  60

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala

                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Ala Ser Arg Val Ala Arg
            260                 265                 270

<210> 61
<211> 270
<212> PRT
<213> Artificial

<220>
<223> Amino acid sequence corresponding to the SLT-1 A1 region with an
example of one N-terminus tag

<400> 61

Lys Gly Met Arg Ser His His His His His His His His Arg Val Ala
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Cys His His His Ala Ser Arg Val Ala Arg
            260                 265                 270


<210>  62
<211>  251
<212>  PRT
<213>  Artificial

<220>
<223>  Amino acid sequence corresponding with the SLT-1 A1 region, with
       cysteine 242 mutated to an alanine

<400>  62

Lys Glu Phe Thr Leu Asp Phe Ser Thr Ala Lys Thr Tyr Val Asp Ser
1               5                   10                  15

Leu Asn Val Ile Arg Ser Ala Ile Gly Thr Pro Leu Gln Thr Ile Ser
            20                  25                  30

Ser Gly Gly Thr Ser Leu Leu Met Ile Asp Ser Gly Ser Gly Asp Asn
        35                  40                  45

Leu Phe Ala Val Asp Val Arg Gly Ile Asp Pro Glu Glu Gly Arg Phe
    50                  55                  60

Asn Asn Leu Arg Leu Ile Val Glu Arg Asn Asn Leu Tyr Val Thr Gly
65                  70                  75                  80

Phe Val Asn Arg Thr Asn Asn Val Phe Tyr Arg Phe Ala Asp Phe Ser
                85                  90                  95

His Val Thr Phe Pro Gly Thr Thr Ala Val Thr Leu Ser Gly Asp Ser
            100                 105                 110

Ser Tyr Thr Thr Leu Gln Arg Val Ala Gly Ile Ser Arg Thr Gly Met
        115                 120                 125

Gln Ile Asn Arg His Ser Leu Thr Thr Ser Tyr Leu Asp Leu Met Ser
    130                 135                 140

His Ser Gly Thr Ser Leu Thr Gln Ser Val Ala Arg Ala Met Leu Arg
145                 150                 155                 160

Phe Val Thr Val Thr Ala Glu Ala Leu Arg Phe Arg Gln Ile Gln Arg
                165                 170                 175

Gly Phe Arg Thr Thr Leu Asp Asp Leu Ser Gly Arg Ser Tyr Val Met
            180                 185                 190

Thr Ala Glu Asp Val Asp Leu Thr Leu Asn Trp Gly Arg Leu Ser Ser
        195                 200                 205

Val Leu Pro Asp Tyr His Gly Gln Asp Ser Val Arg Val Gly Arg Ile
    210                 215                 220

Ser Phe Gly Ser Ile Asn Ala Ile Leu Gly Ser Val Ala Leu Ile Leu
225                 230                 235                 240

Asn Ala His His His Ala Ser Arg Val Ala Arg
                245                 250

<210>  63
<211>  270
<212>  PRT
<213>  Artificial

<220>
<223>  Amino acid sequence corresponding with the SLT-1 A1 region, with
       cysteine 242 mutated to an alanine with an example of one
       N-terminus tag

<400>  63

Lys Gly Met Arg Ser His His His His His His His His Ile Glu Gly
1               5                   10                  15

Arg Ala Ser Lys Glu Phe Thr Leu Asp Phe Ser Thr Ala Lys Thr Tyr
            20                  25                  30

Val Asp Ser Leu Asn Val Ile Arg Ser Ala Ile Gly Thr Pro Leu Gln
        35                  40                  45

Thr Ile Ser Ser Gly Gly Thr Ser Leu Leu Met Ile Asp Ser Gly Ser
    50                  55                  60

Gly Asp Asn Leu Phe Ala Val Asp Val Arg Gly Ile Asp Pro Glu Glu
65                  70                  75                  80

Gly Arg Phe Asn Asn Leu Arg Leu Ile Val Glu Arg Asn Asn Leu Tyr
                85                  90                  95

Val Thr Gly Phe Val Asn Arg Thr Asn Asn Val Phe Tyr Arg Phe Ala
            100                 105                 110

Asp Phe Ser His Val Thr Phe Pro Gly Thr Thr Ala Val Thr Leu Ser
        115                 120                 125

Gly Asp Ser Ser Tyr Thr Thr Leu Gln Arg Val Ala Gly Ile Ser Arg
    130                 135                 140

Thr Gly Met Gln Ile Asn Arg His Ser Leu Thr Thr Ser Tyr Leu Asp
145                 150                 155                 160

Leu Met Ser His Ser Gly Thr Ser Leu Thr Gln Ser Val Ala Arg Ala
                165                 170                 175

Met Leu Arg Phe Val Thr Val Thr Ala Glu Ala Leu Arg Phe Arg Gln
            180                 185                 190

Ile Gln Arg Gly Phe Arg Thr Thr Leu Asp Asp Leu Ser Gly Arg Ser
        195                 200                 205

Tyr Val Met Thr Ala Glu Asp Val Asp Leu Thr Leu Asn Trp Gly Arg
    210                 215                 220

Leu Ser Ser Val Leu Pro Asp Tyr His Gly Gln Asp Ser Val Arg Val
225                 230                 235                 240

Gly Arg Ile Ser Phe Gly Ser Ile Asn Ala Ile Leu Gly Ser Val Ala
                245                 250                 255

Leu Ile Leu Asn Ala His His His Ala Ser Arg Val Ala Arg
            260                 265                 270

<210>  64
<211>  270
<212>  PRT
<213>  Artificial

<220>
<223>  Amino acid sequence corresponding with the SLT-1 A1 region, with
       cysteine 242 mutated to an alanine with an example of one
       N-terminus tag

<400>  64
   Lys Gly Met Arg Ser His His His His His His His His Arg Val Ala
   1               5                   10                  15
   Arg Ala Ser Lys Glu Phe Thr Leu Asp Phe Ser Thr Ala Lys Thr Tyr
               20                  25                  30
   Val Asp Ser Leu Asn Val Ile Arg Ser Ala Ile Gly Thr Pro Leu Gln
           35                  40                  45
   Thr Ile Ser Ser Gly Gly Thr Ser Leu Leu Met Ile Asp Ser Gly Ser
       50                  55                  60
   Gly Asp Asn Leu Phe Ala Val Asp Val Arg Gly Ile Asp Pro Glu Glu
   65                  70                  75                  80
   Gly Arg Phe Asn Asn Leu Arg Leu Ile Val Glu Arg Asn Asn Leu Tyr
                   85                  90                  95
   Val Thr Gly Phe Val Asn Arg Thr Asn Asn Val Phe Tyr Arg Phe Ala
               100                 105                 110
   Asp Phe Ser His Val Thr Phe Pro Gly Thr Thr Ala Val Thr Leu Ser
           115                 120                 125
   Gly Asp Ser Ser Tyr Thr Thr Leu Gln Arg Val Ala Gly Ile Ser Arg
       130                 135                 140
   Thr Gly Met Gln Ile Asn Arg His Ser Leu Thr Thr Ser Tyr Leu Asp
   145                 150                 155                 160
   Leu Met Ser His Ser Gly Thr Ser Leu Thr Gln Ser Val Ala Arg Ala
                   165                 170                 175
   Met Leu Arg Phe Val Thr Val Thr Ala Glu Ala Leu Arg Phe Arg Gln
               180                 185                 190
   Ile Gln Arg Gly Phe Arg Thr Thr Leu Asp Asp Leu Ser Gly Arg Ser
           195                 200                 205
   Tyr Val Met Thr Ala Glu Asp Val Asp Leu Thr Leu Asn Trp Gly Arg
       210                 215                 220
   Leu Ser Ser Val Leu Pro Asp Tyr His Gly Gln Asp Ser Val Arg Val
   225                 230                 235                 240
   Gly Arg Ile Ser Phe Gly Ser Ile Asn Ala Ile Leu Gly Ser Val Ala
                   245                 250                 255
   Leu Ile Leu Asn Ala His His His Ala Ser Arg Val Ala Arg
               260                 265                 270

<210>  65
<211>  21
<212>  DNA
<213>  Artificial

<220>
<223>  DNA sequences of randomly picked clones from the SLT 1 A chain
       tripeptide library. MUC1 epitope

<400>  65
ccagacacgc gaccagctcc a                                                21


<210>  66
<211>  21
<212>  DNA
<213>  Artificial

<220>
<223>  DNA sequences of randomly picked clones from the SLT 1 A chain
       tripeptide library.  Mutant #1

<400>  66
ccagacggga tcggggctcc a                                                21


<210>  67
<211>  21
<212>  DNA
<213>  Artificial

<220>
<223>  DNA sequences of randomly picked clones from the SLT 1 A chain
       tripeptide library.  Mutant #2

<400>  67
ccagacctgg agatggctcc a                                                21


<210>  68
<211>  21
<212>  DNA
<213>  Artificial

<220>
<223>  sequences of randomly picked clones from the SLT 1 A chain
       tripeptide library.  Mutant #3

<400>  68
ccagaccccc gtggggctcc a                                                21


<210>  69
<211>  21
<212>  DNA
<213>  Artificial

<220>
<223>  DNA sequences of randomly picked clones from the SLT 1 A chain
       tripeptide library.  Mutant #4

<400>  69
ccagacgacg acttggctcc a                                                21


<210>  70
<211>  21

<212>  DNA
<213>  Artificial

<220>
<223>  DNA sequences of randomly picked clones from the SLT 1 A chain
       tripeptide library.  Mutant #5

<400>  70
ccagacgtcc ggtgggctcc a                                                21


<210>  71
<211>  21
<212>  DNA
<213>  Artificial

<220>
<223>  DNA sequences of randomly picked clones from the SLT 1 A chain
       tripeptide library. Mutant #6

<400>  71
ccagaccagc gctgggctcc a                                                21


<210>  72
<211>  21
<212>  DNA
<213>  Artificial

<220>
<223>  DNA sequences of randomly picked clones from the SLT 1 A chain
       tripeptide library.  Mutant #7

<400>  72
ccagacctca ggatggctcc a                                                21


<210>  73
<211>  21
<212>  DNA
<213>  Artificial

<220>
<223>  DNA sequences of randomly picked clones from the SLT 1 A chain
       tripeptide library.  Mutant #8

<400>  73
ccagactccc aggaggctcc a                                                21


<210>  74
<211>  21
<212>  DNA
<213>  Artificial

<220>
<223>  DNA sequences of randomly picked clones from the SLT 1 A chain
       tripeptide library.  Mutant #9

<400>  74
ccagactccg accccgctcc a                                                21


<210>  75
<211>  21
<212>  DNA
<213>  Artificial

<220>
<223>  DNA sequences of randomly picked clones from the SLT 1 A chain
      tripeptide library.  Mutant #41

<400>  75
ccagacacgc gccccgctcc a                                                21


<210>  76
<211>  27
<212>  PRT
<213>  Artificial

<220>
<223>  Amino acid sequence alignment of randomly selected clones from
      the SLT 1 A chain tripeptide library.  MUC1 epitope

<400>  76

Cys His His His Pro Asp Thr Arg Pro Ala Pro Ala Ser Arg Val Ala
1               5                   10                  15


Arg Met Ala Ser Asp Glu Phe Pro Ser Met Cys
            20                  25


<210>  77
<211>  27
<212>  PRT
<213>  Artificial

<220>
<223>  Amino acid sequence alignment of randomly selected clones from
      the SLT 1 A chain tripeptide library.  Mutant #1

<400>  77

Cys His His His Pro Asp Gly Ile Gly Ala Pro Ala Ser Arg Val Ala
1               5                   10                  15


Arg Met Ala Ser Asp Glu Phe Pro Ser Met Cys
            20                  25


<210>  78
<211>  27
<212>  PRT
<213>  Artificial

<220>
<223>  Amino acid sequence alignment of randomly selected clones from
      the SLT 1 A chain tripeptide library.  Mutant #2

<400>  78

Cys His His His Pro Asp Leu Gln Met Ala Pro Ala Ser Arg Val Ala
1               5                   10                  15


Arg Met Ala Ser Asp Glu Phe Pro Ser Met Cys
            20                  25


<210>  79
<211>  27
<212>  PRT

<213>  Artificial

<220>
<223>  Amino acid sequence alignment of randomly selected clones from
       the SLT 1 A chain tripeptide library.  Mutant #3

<400>  79

Cys His His His Pro Asp Pro Arg Gly Ala Pro Ala Ser Arg Val Ala
1               5                   10                  15

Arg Met Ala Ser Asp Glu Phe Pro Ser Met Cys
            20                  25

<210>  80
<211>  27
<212>  PRT
<213>  Artificial

<220>
<223>  Amino acid sequence alignment of randomly selected clones from
       the SLT 1 A chain tripeptide library.  Mutant #4

<400>  80

Cys His His His Pro Asp Asp Asp Leu Ala Pro Ala Ser Arg Val Ala
1               5                   10                  15

Arg Met Ala Ser Asp Glu Phe Pro Ser Met Cys
            20                  25

<210>  81
<211>  27
<212>  PRT
<213>  Artificial

<220>
<223>  Amino acid sequence alignment of randomly selected clones from
       the SLT 1 A chain tripeptide library.  Mutant #5

<400>  81

Cys His His His Pro Asp Val Arg Trp Ala Pro Ala Ser Arg Val Ala
1               5                   10                  15

Arg Met Ala Ser Asp Glu Phe Pro Ser Met Cys
            20                  25

<210>  82
<211>  27
<212>  PRT
<213>  Artificial

<220>
<223>  Amino acid sequence alignment of randomly selected clones from
       the SLT 1 A chain tripeptide library.  Mutant #6

<400>  82

Cys His His His Pro Asp Gln Arg Leu Ala Pro Ala Ser Arg Val Ala
1               5                   10                  15

Arg Met Ala Ser Asp Glu Phe Pro Ser Met Cys
            20                  25

<210>  83
<211>  27
<212>  PRT
<213>  Artificial

<220>
<223>  Amino acid sequence alignment of randomly selected clones from
       the SLT 1 A chain tripeptide library.  Mutant #7

<400>  83

Cys His His His Pro Asp Leu Arg Met Ala Pro Ala Ser Arg Val Ala
1               5                   10                  15

Arg Met Ala Ser Asp Glu Phe Pro Ser Met Cys
            20                  25

<210>  84
<211>  27
<212>  PRT
<213>  Artificial

<220>
<223>  Amino acid sequence alignment of randomly selected clones from
       the SLT 1 A chain tripeptide library.  Mutant #8

<400>  84

Cys His His His Pro Asp Ser Gln Glu Ala Pro Ala Ser Arg Val Ala
1               5                   10                  15

Arg Met Ala Ser Asp Glu Phe Pro Ser Met Cys
            20                  25

<210>  85
<211>  27
<212>  PRT
<213>  Artificial

<220>
<223>  Amino acid sequence alignment of randomly selected clones from
       the SLT 1 A chain tripeptide library.  Mutant #9

<400>  85

Cys His His His Pro Asp Ser Asp Pro Ala Pro Ala Ser Arg Val Ala
1               5                   10                  15

Arg Met Ala Ser Asp Glu Phe Pro Ser Met Cys
            20                  25

<210>  86
<211>  27
<212>  PRT
<213>  Artificial

<220>

<223>  Amino acid sequence alignment of randomly selected clones from
       the SLT 1 A chain tripeptide library.  Mutant #41

<400>  86

Cys His His His Pro Asp Thr Arg Pro Ala Pro Ala Ser Arg Val Ala
1               5                   10                  15

Arg Met Ala Ser Asp Glu Phe Pro Ser Met Cys
            20                  25

<210>  87
<211>  7
<212>  PRT
<213>  Artificial

<220>
<223>  MUC-1 sequence

<400>  87

Pro Asp Thr Arg Pro Ala Pro
1               5

<210>  88
<211>  7
<212>  PRT
<213>  Artificial

<220>
<223>  MUC-1 sequence

<220>
<221>  misc_feature
<222>  (3)..(5)
<223>  X is any amino acid

<400>  88

Pro Asp Xaa Xaa Xaa Ala Pro
1               5

## Description

### Background of the Invention

This application relates to libraries of toxin mutants, and to methods of using same in the development of therapeutics targeted against specific cell types.

Plant and bacterial toxins have a structural organization with two or more polypeptide domains or subunits responsible for distinct functions, referred to as A and B. The toxins may be referred to as ABₓ toxins where x represents the number of identical or homologous B subunits in the toxin. This family of framework-related toxins includes examples such as Shiga and Shiga-like toxins, the E. coli heat-labile enterotoxins, cholera toxin, diphtheria toxin, pertussis toxin, Pseudomonas aeruginosa exotoxin A (Olsnes, S. and Sandvik, K. (1988) in hnmunotoxins pp. 39-73, Kluwer Academic, Boston; Sandvik, K., Dubinina, E., Garred, O., et al. (1992) Biochem. Soc. Trans. 20:724) as well as plant toxins such as ricin and abrin. In some cases the toxin are heteromeric, in that the B chains are actually separate entities that connect to the toxic A chain via a non-covalent bonding. In other cases, the toxin is monomeric, since the B chain is part of the same protein when the toxin is produced in nature. In many cases, the A chain has been characterized as having two domains, an A1 domain and an A2 domain.

Based on their ability to block protein synthesis, proteins such as Shiga and Shiga-like toxins as well as ricin, abrin, gelonin, crotin, pokeweed antiviral protein, saporin, momordin, modeccin, sarcin, diphtheria toxin and exotoxin A have been referred to as ribosome-inactivating proteins (RIP). The potency of RIPs is exceedingly high; one molecule of diphtheria toxin A chain (Yamaizumi, et al. (1978) Cell 15:245-250) or ricin A chain (Eiklid, et al. (1980) Exp. Cell Res. 126:321-326) having been shown to be sufficient to kill a eukaryotic cell.

International Patent Publication No. WO99/40185 describes libraries of mutant toxins in which mutations are introduced into the binding domain to alter the type of cells to which the toxic species are delivered. The new proteins are derived by mutating a binding subunit of the wild type heteromeric protein cytotoxic protein to create a library of microorganism clones producing mutant proteins, which are then screened for the ability to specifically bind to and kill a target cell type.

US Patent No. 5,552,144 discloses a Shigella-like toxin II variant to which a mutation is introduced into the A chain at position 167 to change the amino acid at this position to one with a different charge. This resulted in a toxin with less of the enzymatic activity associated with toxicity.

US Patent No. 6,593,132 describes recombinant toxic proteins which are specifically toxic to diseased cells but do not depend for their specificity of action on a specific cell binding component. The recombinant proteins of the '132 patent have an A chain of a ricin-like toxin linked to a B chain by a synthetic linker sequence which may be cleaved specifically by a protease localized in cells or tissues affected by a specific disease to liberate the toxic A chain thereby selectively inhibiting or destroying the diseased cells or tissues.

US Patent No. 6,649,742 discloses Type I ribosome-inactivating proteins (RIPs) and analogs of the RIPs having a cysteine available for disulfide bonding to targeting molecules. The RIPs and RIP analogs are used as components of cytotoxic therapeutic agents to selectively eliminate any cell type to which the RIP component is targeted by the specific binding capacity of the second component of the agent.

PCT application PCT/CA2004/000433 by the present inventors discloses combinatorial protein libraries comprising a plurality of protein species, in which each protein species comprises an A chain of a heteromeric toxic protein, into which an insert has been introduced.

### Summary of the Invention

An aspect of the present invention is a combinatorial protein library comprising a plurality of protein species, each protein species comprising an A chain of a toxic protein into which an insert has been introduced, wherein, (a) the insert is a polypeptide of varying amino acid sequence having a length of at least 2 amino acid residues; and (b) the insert is introduced into the protease-sensitive loop of the A chain sequence. In a further embodiment, the library may comprise up to or over 100 protein species, and may be formed by introducing the insert into a Shiga-like toxin IA chain, for example, between amino acids 242 and 261, such as between amino acids 245 and 246.

In a further embodiment of the present invention, the insert has a length of 7 amino acids.

Another aspect of the present invention is a mutant protein comprising an A chain of a toxic protein into which an insert has been introduced, wherein the insert is a polypeptide of varying amino acid sequence having a length of at least 2 amino acid residues; and the insert is introduced into the protease-sensitive loop of the A chain sequence. In a further embodiment, the A chain can be a Shiga-like toxin I A chain. In a further embodiment, the insert may be introduced between amino acids 242 and 261, such as between amino acids 245 and 246.

A further aspect of the present invention is a method for identifying a ligand that bind to a specific target/receptor, comprising the steps of exposing cells known to possess the target/receptor to members of a combinatorial protein library as described herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

A further aspect of the present invention is a method for isolating a toxin specific for a known target/receptor comprising the steps of: exposing the target/receptor to a combinatorial protein library as described herein; and isolating at least one protein species from the combinatorial protein library captured by binding to the target/receptor.

A further aspect of the present invention is a combinatorial protein library comprising a plurality of protein species, each protein species comprising a modified A chain of a toxic protein into which an insert has been introduced, wherein, the modified A chain comprises a wild-type A chain containing a mutation in at least one cysteine; the insert is a polypeptide of varying amino acid sequence having a length of at least 2 amino acid residues; and the insert is introduced into the region which would be a protease-sensitive loop in the wild-type A chain sequence.

A further aspect of the present invention is a mutant protein comprising a modified A chain of a toxic protein into which an insert has been introduced, wherein, the modified A chain comprises a wild-type A chain containing a mutation in at least one cysteine; the insert is a polypeptide of varying amino acid sequence having a length of at least 2 amino acid residues; and the insert is introduced into a region which would be a protease-sensitive loop in the wild type A chain sequence. In a further embodiment, the modified A chain further comprises a label bound to a cysteine.

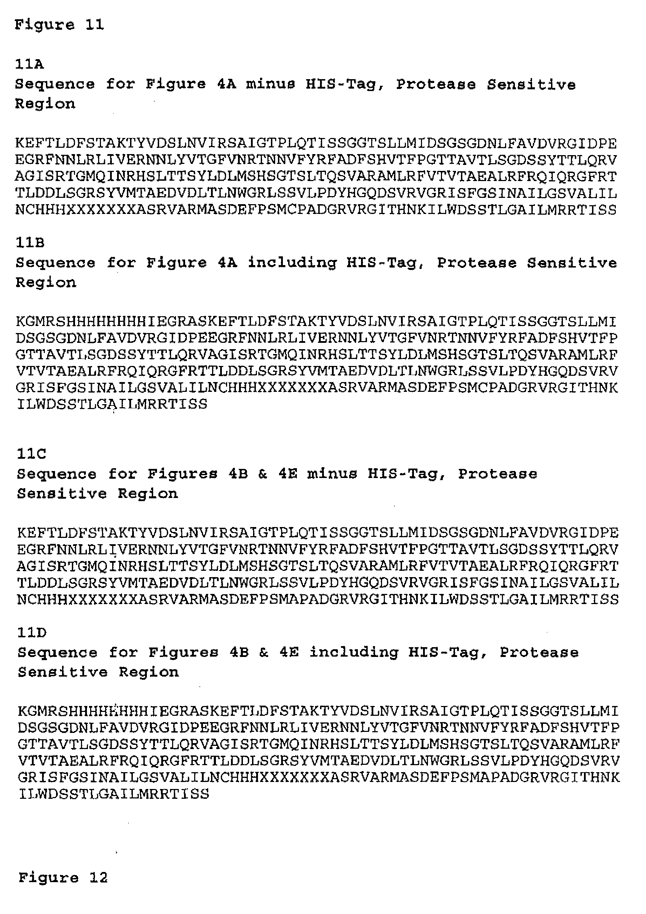

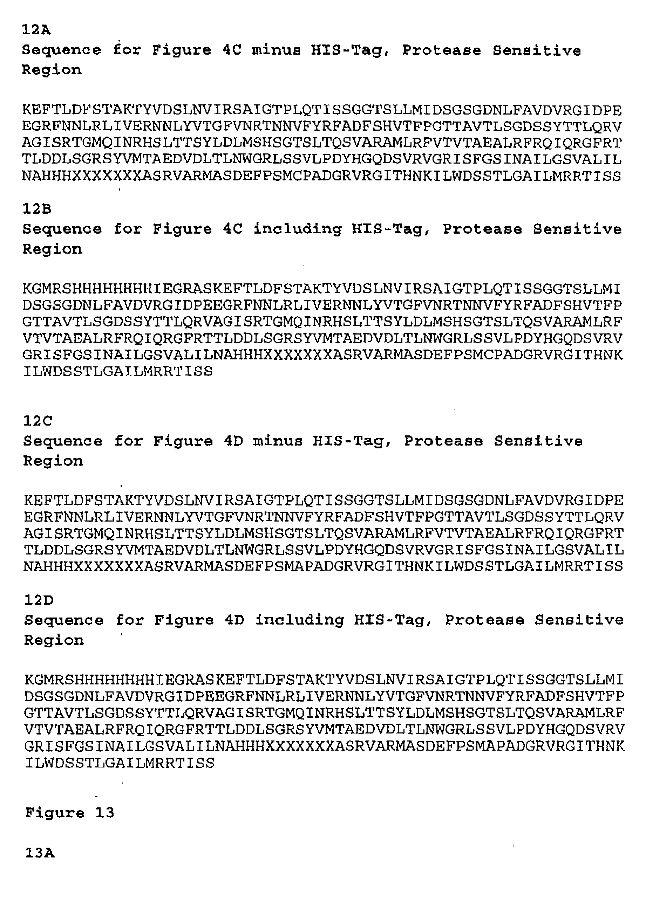

An other embodiment of the present invention is a polypeptide sequence selected from the group consisting of the polypeptide sequence listed in figures 11C, 11D, 12A, 12B, 12C, or 12D.

An other embodiment of the present invention is amethod for identifying a ligand that bind to a specific target/receptor, comprising the steps of: exposing cells known to possess the target/receptor to members of a combinatorial protein library as described herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

An other embodiment of the present invention is a method for isolating a toxin specific for a known target/receptor comprising the steps of: exposing the target/receptor to a combinatorial protein library as described herein; and isolating at least one protein species from the combinatorial protein library captured by binding to the target/receptor.

An other embodiment of the present invention is a combinatorial protein library comprising a plurality of protein species, each protein species comprising an A1 chain of a toxic protein into which an insert has been introduced, wherein, the insert is a polypeptide of varying amino acid sequence having a length of at least 2 amino acid residues; and the insert is introduced into the portion of the A1 chain that would be a protease-sensitive loop if the A1 chain was a full-length A chain.

An other embodiment of the present invention is a mutant protein comprising an A1 chain of a toxic protein into which an insert has been introduced, wherein, the insert is a polypeptide of varying amino acid sequence having a length of at least 2 amino acid residues; and the insert is introduced into the portion of the A1 chain that would be a protease-sensitive loop if the A1 chain was a full length A chain.

Another embodiment of the present invention is a method for identifying a ligand that bind to a specific target/receptor, comprising the steps of: exposing cells known to possess the target/receptor to members of a combinatorial protein library as described herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

Another embodiment of the present invention is a method for isolating a toxin specific for a known target/receptor comprising the steps of: exposing the target/receptor to a combinatorial protein library as disclosed herein; and isolating at least one protein species from the combinatorial protein library captured by binding to the target/receptor.

A further embodiment of the present invention is a combinatorial protein library comprising a plurality of protein species, each protein species comprising a modified A1 chain of a toxic protein into which an insert has been introduced, wherein, the modified A1 chain comprises a wild-type A1 chain containing a mutation in one cysteine; the insert is a polypeptide of varying amino acid sequence having a length of at least 2 amino acid residues; and the insert is introduced into the portion of the A1 chain that would be a protease-sensitive loop if the A1 chain was a full length A chain.

A further embodiment of the present invention is a mutant protein comprising a modified A1 chain of a toxic protein into which an insert has been introduced, wherein, the modified A1 chain comprises a wild-type A1 chain containing a mutation in one cysteine; the insert is a polypeptide of varying amino acid sequence having a length of at least 2 amino acid residues; and the insert is introduced into the portion of the A1 cahin that would be a protease-sensitive loop if the A1 chain was a full-length A chain.

A further embodiment of the present invention is a method for identifying a ligand that bind to a specific target/receptor, comprising the steps of: exposing cells known to possess the target/receptor to members of a combinatorial protein library as disclosed herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

A further embodiment of the present invention is a method for isolating a toxin specific for a known target/receptor comprising the steps of: exposing the target/receptor to a combinatorial protein library as disclosed herein; and isolating at least one protein species from the combinatorial protein library captured by binding to the target/receptor.

In another aspect of the present invention, the combinatorial protein library can have the insert introduced at the N-terminus of the A chain sequence instead of in the protease sensitive loop region.

Another embodiment of the present invention is a method for identifying a ligand that bind to a specific target/receptor, comprising the steps of: exposing cells known to possess the target/receptor to members of a combinatorial protein library as disclosed herein; selecting members of the protein library which are observed to be toxic to the cells;evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

An other embodiment of the present invention is a method for isolating a toxin specific for a known target/receptor comprising the steps of: exposing the target/receptor to a combinatorial protein library as disclosed herein; and isolating at least one protein species from the combinatorial protein library captured by binding to the target/receptor.

### Brief Description of the Drawings

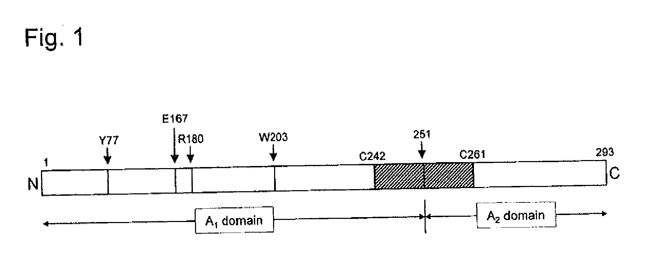

Fig. 1 is a schematic diagram depicting the A1 and A2 domains of wild-type SLT-1. The A chain is composed of 293 amino acids. The chain is cleaved by furin to produce an A1 catalytic fragment and an A2 C-teminal tail non-covalently associated with the B pentamer. A protease-sensitive loop (stripped area) is defined by the only two cysteine residues in the A chain (Cys 242 and 261). Tyr77, Glu167, Arg170 and Trp203 represent residues important for the catalytic activity of the A1 domain (arrows).

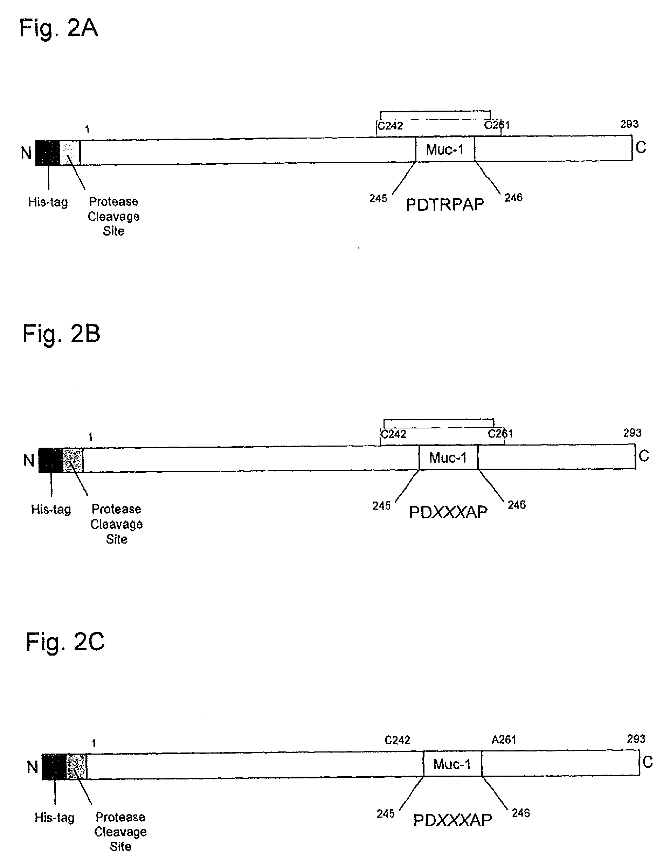

Fig. 2A is a schematic representation of the SLT-1 A chain (1-293) with the breast cancer-associated MUC1 epitope PDTRPAP (control sequence recognized by the mAb One M27) inserted between residues 245 and 246 and a 6-Histidine tag followed by a protease cleavage site (such as a furin or thrombin cleavage site) at its N-terminus.
Fig. 2 B is a depiction of a SLT-1 A chain-tripeptide library construction where the three positions of the MUC1 epitope recognized by the mAb One M27 are randomized (XXX region). The tripeptide library was inserted in a naturally occurring loop region of the A chain created by the presence of a disulfide bridge between Cys 242 and Cys 261.
Fig. 2C is a depiction of the SLT-1 A chain tripeptide library construction of Figure 2B, wherein cysteine 261 has been mutated to an alanine. Note that in this case (as in many of the figures below) the disulfide bridge present in Fig. 2B can not be formed due to the absence of one of the cysteines.

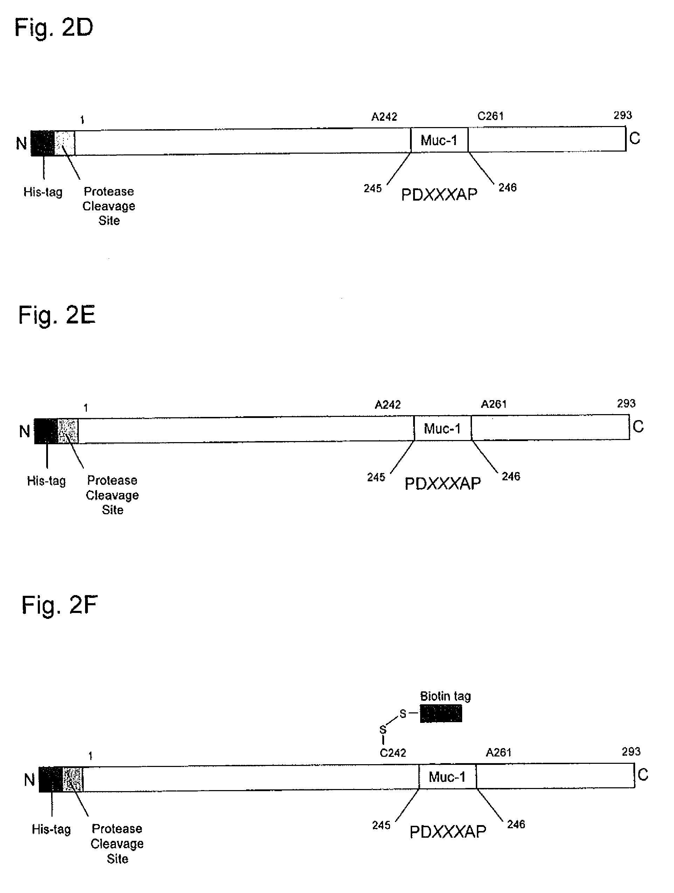

Figure 2D is a depiction of the SLT-1 A chain tripeptide library construction of Figure 2B, wherein cysteine 242 has been mutated to an alanine.
Figure 2E is a depiction of the SLT-1 A chain tripeptide library construction of Figure 2B, wherein cysteine 242 and cysteine 261 have both been mutated to alanine residues.
Figure 2F is a depiction of the SLT-1 A chain tripeptide library construction of Figure 2C, wherein the C242 residue has been biotinylated.

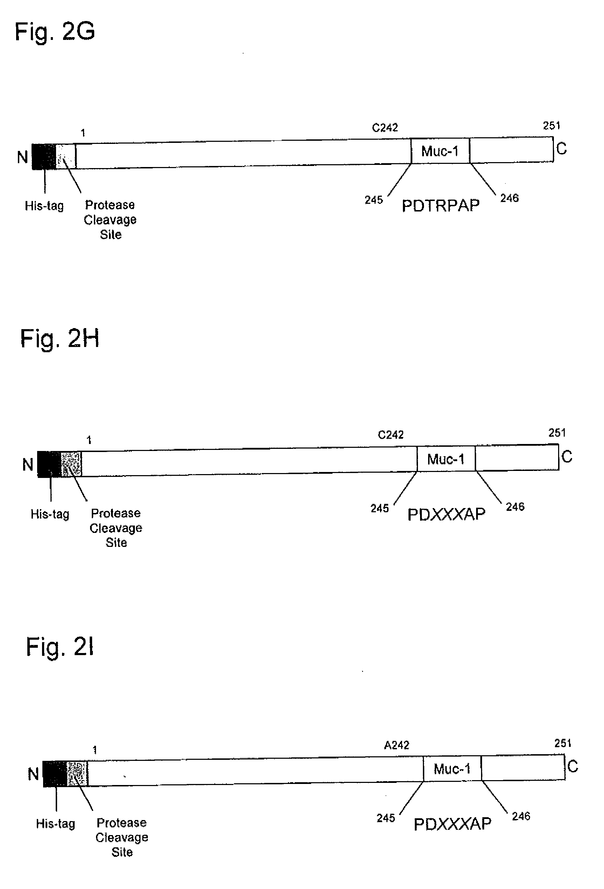

Figure 2G is a schematic representation of the SLT-1 A1 chain (1-251) with the breast cancer-associated MUC 1 epitope PDTRPAP (control sequence recognized by the mAb One M27) inserted between residues 245 and 246 and a Histidine tag followed by a protease cleavage site (such as a furin or thrombin cleavage site) at its N-terminus.
Fig. 2 H is a depiction of our SLT-1 A1 chain-tripeptide library construction where the three key positions of the MUC1 epitope recognized by the mAb One M27 were randomized (XXX region).
Fig. 2 I is a depiction of the SLT-1 A1 chain tripeptide library construction of Figure 2H, wherein cysteine 242 has been mutated to an alanine.
Figure 2J is a depiction of the SLT-1 A1 chain tripeptide library construction of Figure 2H, wherein cysteine 242 has been biotinylated.
Fig. 3 shows a representative ELISA data set from screening 96 distinct single A chain variants from our SLT-1 A chain-tripeptide library with mAb One M27. Toxin variant # 41 (Tables 1 and 2) gave a strong ELISA signal and had the expected epitope.
Fig. 4A shows a schematic diagram of a 7-amino acid random segment inserted between residues 245 and 246 of the A chain.
Fig. 4B shows a schematic diagram of a 7-amino acid random segment inserted between residues 245 and 246 of the A chain, wherein the cysteine at position 261 has been mutated to an alanine.
Fig. 4C shows a schematic diagram of a 7-amino acid random segment inserted between residues 245 and 246 of the A chain, wherein the cysteine at position 242 has been mutated to an alanine.
Fig. 4D shows a schematic diagram of a 7-amino acid random segment inserted between residues 245 and 246 of the A chain, wherein the cysteine at positions 242 and 261 have been mutated to alanines.
Fig. 4E shows a schematic diagram of a 7-amino acid random segment inserted between residues 245 and 246 of the A chain, wherein the cysteine at position 261 has been mutated to an alanine and the cysteine at position 242 has been biotinylated.
Fig. 4F shows a schematic diagram of a 7-amino acid random segment inserted between residues 245 and 246 of the A1 chain.
Fig. 4G shows a schematic diagram of a 7-amino acid random segment inserted between residues 245 and 246 of the A1 chain, wherein the cysteine residue at position 242 has been biotinylated.
Fig. 4H shows a schematic diagram of a 7-amino acid random segment inserted between residues 245 and 246 of the A1 chain, wherein the cysteine residue at position 242 has been mutated to an analine.
Fig. 5 shows the results of tests on seven toxin variants that were identified as repeatable killers of the human melanoma cell line 518A2. The abscissa represents the log concentration of toxin used to treat the cells and the ordinate depicts the observed percentage of cells that are viable after 48 hours. The closed triangles depicts the effect of the wild type toxin on 518A2 cells while the two most efficacious A chain variants were termed SAM#3 (open squares) and SAM#5 (X symbols).
Fig. 6 shows the amino acid sequence corresponding to the wild-type SLT-1 protein of Figure 1. Amino acid sequences are shown from N-terminus to C-terminus.
Figure 7A shows the Amino acid sequence corresponding to the SLT-A chain of Figure 2A, less the N-terminus tags (the His-tag and the protease cleavage site).
Figure 7B shows the amino acid sequence corresponding to the SLT-A chain of Figure 2A, with an example of one possible N-terminus tag, comprising a 8-His-tag and a protease cleavage site.
Figure 7C shows the amino acid sequence corresponding to the SLT-A chain-tripeptide library construction of Figure 2B, less the N-tenninus tags (the His-tag and the protease cleavage site).
Figure 7D shows the amino acid sequence corresponding to the SLT-A chain-tripeptide library construction of Figure 2B, with an example of one possible N-terminus tag, comprising a 8-His-tag and a protease cleavage site.
Figure 8A shows the amino acid sequence corresponding to the SLT-1 A chain tripeptide library of Figure 2C and 2F, less the N-terminus tags (the His-tag and the protease cleavage site).
Figure 8B shows the amino acid sequence corresponding to the SLT-1 A chain tripeptide library of Figure 2C and 2F, with an example of one possible N-terminus tag, comprising a 8-His-tag and a protease cleavage site.
Figure 8C shows the amino acid sequence corresponding to the SLT-1 A chain tripeptide library of Figure 2D, less the N-terminus tags (the His-tag and the protease cleavage site).
Figure 8D shows the amino acid sequence corresponding to the SLT-1 A chain tripeptide library of Figure 2D, with an example of one possible N-terminus tag, comprising a 8-His-tag and a protease cleavage site.
Figure 9A shows the amino acid sequence corresponding to the SLT-1 A chain tripeptide library of Figure 2E, less the N-terminus tags (the His-tag and the protease cleavage site).
Figure 9B shows the amino acid sequence corresponding to the SLT-1 A chain tripeptide library of Figure 2E, with an example of one possible N-terminus tag, comprising a 8-His-tag and a protease cleavage site.
Figure 9C shows the amino acid sequence corresponding to the SLT-1 A1 chain of Figure 2G, less the N-terminus tags (the His-tag and the protease cleavage site).
Figure 9D shows the amino acid sequence corresponding to the SLT-1 A1 chain of Figure 2G, with an example of one possible N-terminus tag, comprising a 8-His-tag and a protease cleavage site.
Figure 10A shows the amino acid sequence corresponding to the SLT-1 A1 chain of figure 2H and 2J, less the N-terminus tags (the His-tag and the protease cleavage site).
Figure 10B shows the amino acid sequence corresponding to the SLT-1 A1 chain of figure 2H and 2J, with an example of one possible N-terminus tag, comprising a 8-His-tag and a protease cleavage site.
Figure 10C shows the amino acid sequence corresponding to the SLT-1 A1 chain of figure 2I, less the N-terminus tags (the His-tag and the protease cleavage site).
Figure 10D shows the amino acid sequence corresponding to the SLT-1 A1 chain of figure 2I, with an example of one possible N-terminus tag, comprising a 8-His-tag and a protease cleavage site.
Figure 11A shows the amino acid sequence corresponding to the SLT-1 A chain of Figure 4A, less the N-terminus tags (the His-tag and the protease cleavage site).
Figure 11B shows the amino acid sequence corresponding to the SLT-1 A chain of Figure 4A, with an example of one possible N-terminus tag, comprising a 8-His-tag and a protease cleavage site.
Figure 11C shows the amino acid sequence corresponding to the SLT-1 A chain of Figure 4B and 4E, less the N-terminus tags (the His-tag and the protease cleavage site).
Figure 11D shows the amino acid sequence corresponding to the SLT-1 A chain of Figure 4B and 4E, with an example of one possible N-terminus tag, comprising a 8-His-tag and a protease cleavage site.
Figure 12A shows the amino acid sequence corresponding to the SLT-1 A chain of Figure 4C, less the N-terminus tags (the His-tag and the protease cleavage site).
Figure 12B shows the amino acid sequence corresponding to the SLT-1 A chain of Figure 4C, with an example of one possible N-terminus tag, comprising a 8-His-tag and a protease cleavage site.
Figure 12C shows the amino acid sequence corresponding to the SLT-1 A chain of Figure 4D, less the N-terminus tags (the His-tag and the protease cleavage site).
Figure 12D shows the amino acid sequence corresponding to the SLT-1 A chain of Figure 4D, with an example of one possible N-terminus tag, comprising a 8-His-tag and a protease cleavage site.
Figure 13A shows the amino acid sequence corresponding to the SLT-1 A1 chain of Figure 4F and 4G, less the N-terminus tags (the His-tag and the protease cleavage site).
Figure 13B shows the amino acid sequence corresponding to the SLT-1 A1 chain of Figure 4F and 4G, with an example of one possible N-terminus tag, comprising a 8-His-tag and a protease cleavage site.
Figure 13C shows the amino acid sequence corresponding to the SLT-1 A1 chain of Figure 4H, less the N-terminus tags (the His-tag and the protease cleavage site).
Figure 13D shows the amino acid sequence corresponding to the SLT-1 A1 chain of Figure 4H, with an example of one possible N-terminus tag, comprising a 8-His-tag and a protease cleavage site.
Figure 14A shows the amino acid sequence corresponding to the SLT-1 A region, with cysteine 2G1 mutated to an alanine.
Figure 14B shows the amino acid sequence corresponding to the SLT-1 A region, with cysteine 261 mutated to an alanine, and with an example of one possible N-terminus tag, comprising an 8-HIS-tag and a protease cleavage site.
Figure 14C shows the amino acid sequence corresponding to the SLT-1 A region, with cysteine 242 mutated to an alanine.
Figure 14D shows the amino acid sequence corresponding to the SLT-1 A region, with cysteine 242 mutated to an alanine, and with an example of one possible N-terminus tag, comprising an 8-HIS-tag and a protease cleavage site.
Figure 15A shows the amino acid sequence corresponding to the SLT-1 A region, with cysteines 242 and 261 mutated to alanines.
Figure 15B shows the amino acid sequence corresponding to the SLT-1 A region, with cysteines 242 and 261 mutated to alanines, and with an example of one possible N-terminus tag, comprising an 8-HIS-tag and a protease cleavage site.
Figure 15C shows the amino acid sequence corresponding to the SLT-1 A1 region.
Figure 15D shows the amino acid sequence corresponding to the SLT-1 A1 region with an example of one possible N-terminus tag, comprising an 8-HIS-tag and a protease cleavage site.
Figure 16A shows the amino acid sequence corresponding to the SLT-1 A1 region, with cysteine 242 mutated to an alanine.
Figure 16B shows the amino acid sequence corresponding to the SLT-1 A1 region with cysteine 242 mutated to an alanine, and with an example of one possible N-terminus tag, comprising an 8-HIS-tag and a protease cleavage site.

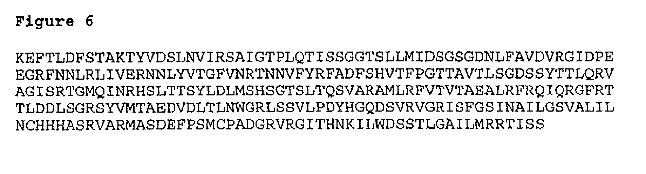

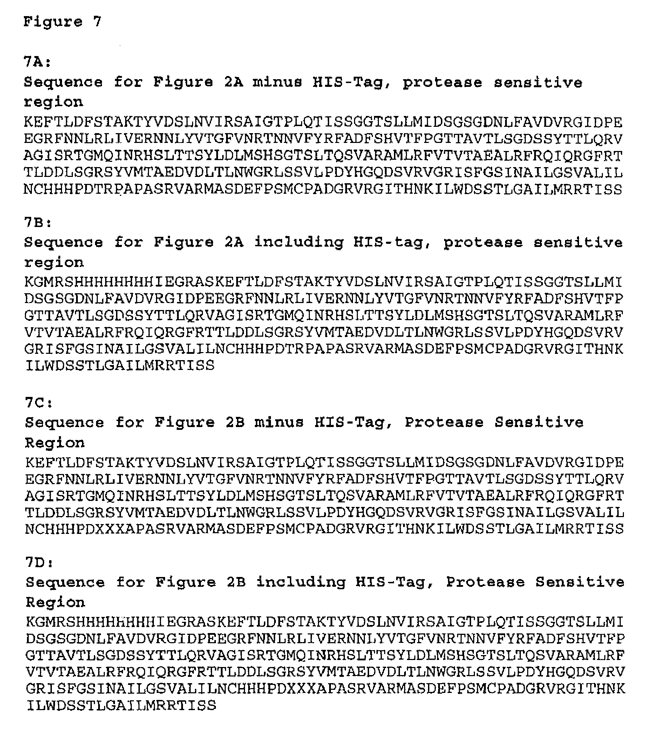

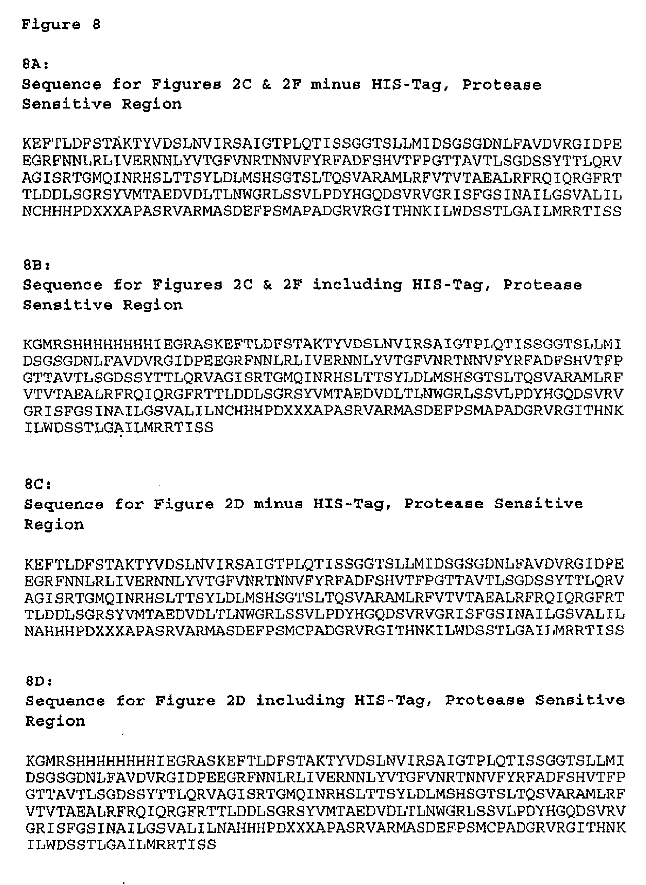

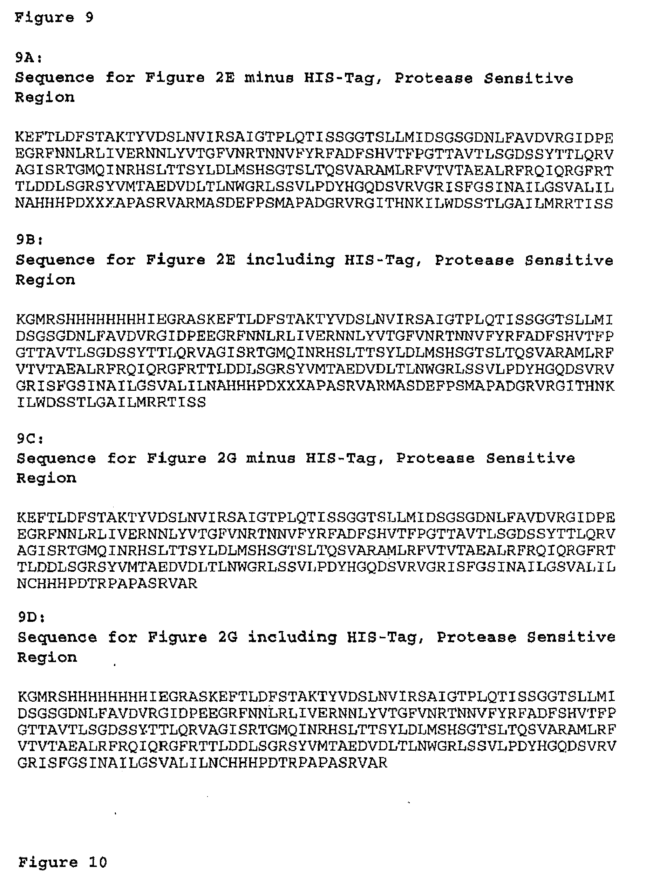

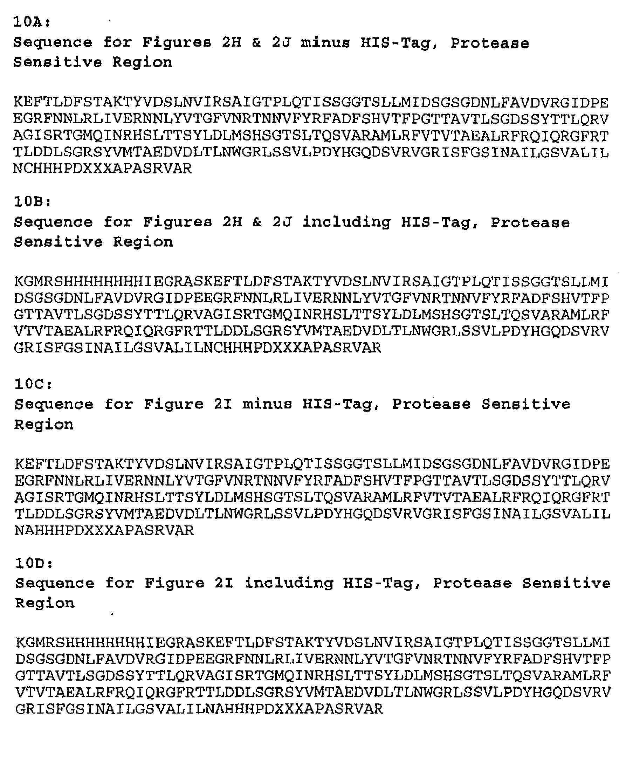

For figures 6-16 that include the N-terminus tag, the tag can be varied as needed; for example, the tag KGMRSHHHHHHHHRVARAS can be used instead of KGMRSHHHHHHHHIEGRAS.

### Detailed Description of the Invention

The present invention relates to a combinatorial protein library comprising a plurality of protein species, each protein species comprising an A or A1 chain of a heteromeric toxic protein into which an insert has been introduced. The insert is a polypeptide of varying amino acid sequence having a length of 2 or more amino acid residues, for example from 3 to 200 amino acid residues; and is introduced into the protease-sensitive loop of the A chain sequence, or the equivalent remaining area in the A1 chain sequence. The library provides a collection of protein species that can be screened for individual proteins that are toxic against specific cell types, such as specific cancer cell types. Individual protein species thus selected are suitably used in the treatment of the cancer.

As used in the specification and claims of this application, the term "combinatorial library" refers to a mixture of species each of which has a common portion and a variable portion. In the case of a "combinatorial protein library" each of the species is a protein or peptide, and the common portions and the variable portions are each amino acid sequences. In the case of a combinatorial expression library, the species are microorganisms, expression vectors or polynucleotides which, when expressed, produce proteins or peptides having common portions and variable portions. In this case, the common portions and the variable portions are each nucleotide sequences. Since the purpose of the combinatorial library is to provide multiple variants for screening purposes, the combinatorial library preferably contains at least 100 distinct species of protein or expression unit, more preferably at least 1000 distinct species.

As used in the specification and claims of this application, the term "heteromeric toxic protein" refers to the class of protein toxins with the common organization theme of being heteromeric in nature with two or more polypeptide domains or subunits responsible for distinct functions (Merritt, E.A., and Hol, W.G.J. (1995) Curr. Opin. Struct. Biol. 5:165 1). In such proteins, the two or more subunits or domains could be referred to as A and B, and the toxins as ABₓ toxins where x represents the number of identical or homologous B subunits in the toxin. This family of framework-related toxins includes examples such as Shiga and Shiga-like toxins, the E. coli heat-labile enterotoxins, cholera toxin, diphtheria toxin, pertussis toxin, Pseudomonas aeruginosa exotoxin A as well as plant toxins such as ricin and abrin. Based on their ability to block protein synthesis, proteins such as Shiga and Shiga-like toxins as well as ricin, abrin, gelonin, crotin, pokeweed antiviral protein, saporin, momordin, modeccin, sarcin, diphtheria toxin and exotoxin A have been referred to as ribosome-inactivating proteins (RIP). In these naturally-occurring heteromeric toxic proteins, the A chain is the toxic portion, while the B chains form a binding moiety which binds to a receptor on a cell susceptible to the toxin, thereby delivering the A chain to the cell. In many cases, the A chain has been further characterized as having two domains, an A1 domain and an A2 domain. Typically, the A1 domain includes the cytotoxic elements of the A subunit and a portion of the protease-sensitive loop portion, but often does not include the B chain binding portion.

One specific example of the A chain of a heteromeric toxic protein is the A chain of SLT-1 which has the sequence given is Seq. ID No. 1. The A chain of SLT-1 comprises of 293 amino acids with the enzymatic (toxic) domain spanning residues 1 to 239. A protease sensitive loop encompassing residues 242 to 261 is normally exposed, and is a suitable site for inserting a peptide sequence. The A chain of SLT-1 is characterized as having an A1 domain, spanning residues 1 to 251, and including the entire enzymatic (toxic) domain, and an A2 domain spanning residues 252-261.

SLT-1 is a type II ribosome inactivating protein produced by pathogenic strain of Escherichia coli (0157:H7) (24). SLT-1 is an AB5 complex of about 70 kD (O'Brien, A. D., and Holmes, R. K. (1987) Shiga and Shiga-like toxins. Microbiol Rev 51, 206-220.). The single 32 kD catalytic A subunit is non-covalently associated with a pentamer of five identical 7.7 kD B subunits. The B subunit pentamer recognizes the glycolipid globotriaosylceramide (also known as CD77 or Gb3) on the surface of target cells (Lingwood, C. A. (1993) Verotoxins and their glycolipid receptors. Adv Lipid Res 25, 189-211; Jacewicz, et al. (1986) Pathogenesis of shigella diarrhea. XI. Isolation of a shigella toxin-binding glycolipid from rabbit jejunum and HeLa cells and its identification as globotriaosylceramide. J Exp Med 163, 1391-1404). A protease-sensitive loop located between Cys242 and 261 at the C terminus of the A chain is cleaved by furin during cellular routing (Fig. 1). The A chain remains associated with its B subunit pentamer due to an intrachain disulfide bond between Cys242 and Cys261 as it travels to the ER lumen (Sandvig, et al. (1989) Endocytosis from coated pits of Shiga toxin: a glycolipid-binding protein from Shigella dysenteriae 1. J Cell Biol 108, 1331-1343;32. Garred, et al. (1995) Role of processing and intracellular transport for optimal toxicity of Shiga toxin and toxin mutants. Exp Cell Res 218, 39-49.). The disulfide bond is finally reduced in the ER lumen and the A1 chain (first 251 aa) is released and subsequently retrotranslocated to the cytosol where it inactivates ribosomes (O'Brien, et al. (1992) Shiga toxin: biochemistry, genetics, mode of action, and role in pathogenesis. Curr Top Microbiol Immunot 180, 65-94.). More specifically, the A chain of SLT-1 is a N-glycosidase that catalytically cleaves a specific adenine nucleotide (4324) from the 28 S rRNA (Brigotti, et al. (1997) The RNA-N-glycosidase activity of Shiga-like toxin I: kinetic parameters of the native and activated toxin. Toxicon 35, 1431-1437.). This event leads to the inhibition of protein synthesis by preventing the binding of aminoacyl tRNAs to the ribosome and halting protein elongation. Mutagenesis studies as well as structural analysis performed on the A chains of ST and ricin have delineated key conserved residues involved in catalytic activity (Deresiewicz, et al.(1992) Mutations affecting the activity of the Shiga-like toxin I A-chain. Biochemistry 31, 3272-3280; Ready, et al. (1991) Site-directed mutagenesis of ricin A-chain and implications for the mechanism of action. Proteins 10, 270-278). Residues important for catalytic activity of SLT-1 are tyrosine 77, glutamic acid 167, arginine 170 and tryptophan 203 (Hovde, et al. (1988) Evidence that glutamic acid 167 is an active-site residue of Shiga-like toxin I. Proc Natl Acad Sci U S A 85, 2568-2572; Yamasaki, et al. (1991) Importance of arginine at position 170 of the A subunit of Vero toxin 1 produced by enterohemorrhagic Escherichia coli for toxin activity. Microb Pathog 11, 1-9). In addition, binding of the toxin to the cell surface is important to introduction into the cell and thus for toxic activity. Because of this the A chain alone is not significantly toxic.

In addition to the A or A1 chain of SLT-1, other toxins may also be used to form libraries and compositions in accordance with the invention, and may be used in the methods of the invention. Specifically, Shiga and other Shiga-like toxins, as well as ricin, abrin, gelonin, crotin, pokeweed antiviral protein, saporin, momordin, modeccin, sarcin, diphtheria toxin and exotoxin A, and other functionally-related ribosome inactivating proteins (RIP) may be used.

For purposes of making the combinatorial library of the invention, short amino acid sequences of at least 2 amino acids, for example of 3 to 200 amino acids residues in length, are inserted into this protease sensitive loop. The number of amino acids in the insert defines the number of possible random variants that can be in the library. For example, when the number of amino acids in the insert is 3, the maximum number of variants is 20³ or 8000 variants. Larger inserts provide corresponding larger numbers of possible variants. It has also been found that certain other regions of the A or A1 chain maybe used to insert the short amino acid sequences. The present inventors have shown, for example, that the N terminus region can support insertions of long amino acid sequences without significant loss of activity.

As an alternative to using inserts with purely random sequences, inserts can be designed based on a known template. For example, as described below in the context of Muc-1, variations in a sequence known to provide receptor binding properties for a particular cell type can be used to identify an insert that provides for optimization of the toxic properties of the protein construct. This same optimization may be performed on an individual sequence isolated by screening of a larger combinatorial library. It will be appreciated, however, that the insert in the proof of principle tests of Example 1 use an insert which is the target/receptor, while in the actual case the insert would be based on the sequence of a known ligand to be optimized for maximum effectiveness and specificity.

This approach, in which a specific, known receptor type is targeted illustrates a further aspect of the invention, namely a method for identifying peptide ligands that bind to specific targets/receptors, such as tumor markers known to exist on cancer cells. In this method, a combinatorial protein library in accordance with the invention is screened against cells known to possess the target/receptor. The toxin serves as the reporter, such that proteins which are shown to be toxic to the cells are evaluated to determine the sequence of the inserted region. Peptides of this sequence can then be used, in combination with a toxin or other molecules, to direct compounds to cells possessing the target/receptor. Further testing peptides of the sequence of the inserted region may be appropriate to confirm that they are a ligand for the specific target/receptor, as opposed to some other receptor on the cell type. This can be done using binding assays with isolated receptor, where such are available.

These inserts used in alternative to inserts with purely random sequences can use, as a known template, a small protein molecule with a known or desirable folded structure, for increasing sensitivity or specificity of binding. For example, an immunoglobulin like domain, such as a FC molecule sequence, a fragment of an FC molecule sequence, or a mimic of an FC molecule sequence, can be inserted. Libraries can therefore be created with a variable chain domain region of an FC molecule, or a random 7-mer (or other length random sequence) inserted in the variable region of an FC molecule, in turn inserted in the protease-sensitive loop region of the A or A1 molecule.

The invention also provides a method for identifying toxic substances specific for a known cell marker, and particularly markers that are available in isolated form. In this embodiment of the invention, the toxin need not serve as reporter. Thus, cells having the marker, or an isolated target/receptor, where available, are exposed to the combinatorial protein library. In preferred embodiments, the cells or the isolated target/receptor are immobilized on a solid support, such as in plastic wells. Captured proteins from the library are then rescreened against cells to confirm their toxicity and specificity for cells expressing the target/receptor, and their suitability for use as a therapeutic. This method can be used to identify toxins with binding inserts specific for any tumor marker or cell receptor, including without limitation tumor markers such as mucins such as MUC-1 and its glycoforms, Her-2, Her2-Neu, tyrosine kinase markers, EGFR, GD2, and GD3.

Thus, in accordance with this specific aspect of the invention, a method for isolating a toxin specific for a known target/receptor is provided that comprises the steps of:
(a) exposing the target/receptor to a combinatorial protein library comprising a plurality of protein species, each protein species comprising an A or A1 chain of a toxic protein into which an insert has been introduced, wherein,
   the insert is a polypeptide of varying amino acid sequence having a length of at least 2 amino acid residues; and
   the insert is introduced into the protease-sensitive loop of the A chain sequence or the equivalent portion of the A1 chain sequence; and
(b) isolating at least one protein species from the combinatorial protein library captured by binding to the target/receptor. As used in the specification and claims hereof, the term "isolating" refers to any mechanism for obtaining a composition containing the protein separated from the milieu in which it is expressed in a form suitable for further analysis. This would include release from the target/receptor following capture (for example by exposure to a competitive binding agent) or isolation from a culture of a clone expressing the protein found to be captured. The method may further comprises the step of screening the isolated protein against cells expressing the target/receptor, to confirm their toxicity for cells expressing the target/receptor. Procedures suitable for this screening are described in Example 3. As noted above, in this method, the target/receptor may be a purified target/receptor and may be immobilized on a solid support. The target/receptor may also be on the surface of cells, which may be immobilized. Where the target/receptor is on the surface of cells, the toxin can serve as a reporter, and the death of the cells is indicative of receptor binding.

Our experience with constructing SLT-1 libraries has pointed out a number of practical issues that are appropriately considered in selecting a protein template for building combinatorial libraries. An important factor is to choose a single chain protein, preferably a bacterial protein of less than 300 amino acids (if the libraries are to be expressed in prokaryotes). Use of a smaller toxin both increases its potential to penetrate into solid tumors and reduces its immunogenicity. Secondly, the protein template should spontaneously fold in solution into its active form such that there is minimal need for host's chaperones. One should avoid for example scaffolds that contain multiple cysteine residues normally involved in disulfide bridges. In addition, a single chain protein as opposed to a multi-subunit complex may be more easily exported from bacteria. Thirdly, the protein template should possess an enzymatic activity, which can rapidly be measured to confirm the proper folding of peptide variants containing single and multiple site-directed mutations. Fourthly, a simple screening approach should be incorporated into the design of combinatorial libraries. Such searches should be amenable to high-throughput screening methods. The catalytic A chain of SLT-1 (residues 1 to 293) and the A1 chain of SLT-1 (residues 1-251) meet these criteria because they are a single chain that lack any known receptor binding function, and that have well defined structures and catalytic sites.

We have also found that cysteine 242 and 261 of the A chain of SLT-1 can be replaced by site specific mutagenesis (or otherwise modified) in order to remove the single disulfide bridge present between these two cysteines in the A chain. Such advantageous mutations remove the need to reduce the peptide chain during purification and provide a simpler strategy for purifying single chain toxins comprising the A1 domain of A chain mutants. We have found that either cysteine 242 or cysteine 261, or both, can be mutated in this manner. The cysteine(s) can be mutated to alanine, though other mutations may result in the same desired effect while providing a suitable A or A1 chain.

An other advantage of mutating the cysteines in this manner results from the mutation of either of C242 or C261 (preferably, C261). This results in a shiga-like protein A chain with only one cysteine. The thiol group associated with this single cysteine residue is therefore free and offers the opportunity to introduce spectroscopic (derivatives of fluorescent chromophores that react with the thiol moiety), radioactive (thiol-reactive heavy ion complexes or metal chelators), or biochemical (for example, biotin) probe. These A chain variants, labeled with reporter probes, can then be used as agents for both in vitro and in vivo applications. For example, an inactivated single chain A or A1 variant, labeled with a reporter probe (and inactivated by mutating E167A and R170A) can be used as a non-toxic probe, as described above, to determine which patient population may benefit from being treated with which of the corresponding catalytically active single chain variants. A library of inactivated single chain A1 variants labeled in this manner can also be used to determine which binding domains (from said library) are effective, to determine which binding domains can be used in corresponding catalytically active single chain variants to treat a patient. Alternatively, these non-toxic probes can be used to determine which binding domains (from the randomly mutated library) can be used to target the desired cell population and used in either the toxic subunit, or bound to a separate and known therapeutic agent, to target and treat that cell population. Alternatively, once a specific desired library member has been identified, either through binding studies as described above or otherwise, that one specific library member (for example, an A1 chain with a specific desirable 7mer sequence inserted after amino acid 245) can be biotinylated for further purification or other studies.

Alternatively, the entire library can be biotinylated, allowing the library to be assayed with a receptor based assay rather than a toxin based assay.

We have also surprisingly found that the use of the A1 domain of the A chain is sufficient for performing the assays described herein. Although the A1 domain contains the entirety of the cytotoxic domain of the A subunit, it was not known until know what function was served by the A2 subunit in assays of this kind, and whether the A1 subunit could be succesfully used on its own in this type of assay. By removing the A2 portion of the A chain, a smaller, more easily manipulated fragment can be used in the assays described herein.

Combining the unexpected finding that cysteine mutations result in a functional A subunit useful in the assays described herein, with the finding that the A1 subunit can be used alone yields the further advantage of combining these two findings. By mutating one of the cysteines (for example, C261), one can make a protein that can not form the protease-sensitive A chain loop (since this protease-sensitive A chain loop is a result of the cysteine-cysteine interactions between C242 and C261). The A subunit can therefore be cleaved more efficiently into A1 and A2 subunits. Thus mutation of at least one of C242 and C261, combined with utilizing the A1 subunit alone, provides a simpler and more elegant strategy for purifying single chain toxins comprising the A1 domain of A chain mutants. Manufacturing, and purifying, a protein or a protein library of use in the assays described herein thus becomes much more efficient by either utilizing the A1 domain alone, at least one cysteine mutation, or both.

A further aspect of the invention is a combinatorial expression library comprising a plurality of species of expression systems. Each species within the expression library expresses a protein species comprising an A or A1 chain of a heteromeric toxic protein into which an insert has been introduced. The insert is a polypeptide of varying amino acid sequence having a length of 2 or more amino acid residues, for example from 3 to 200 amino acid residues; and is introduced into the protease-sensitive loop of the A chain sequence. Suitable expression systems include plasmids and viral vectors.

In a further aspect of the invention, the A or A1 chain can be pegylated or otherwise modified by a method known in the art in order to stabilize or modify the biological half-life of the protein. Reactive pegylation agents can be bound to a lysine, to the N-terminus, or to cysteine 242 or 261.

We have also found that adding a histidine tag (for example, a 6 or 8 histidine tag) to the N-terminus of the A or A1 chain is advantageous as it allows for improved purification of the desired protein. Optionally, a protease-sensitive region can be inserted between the histidine tag and the rest of the A or A1 chain in order to facilitate cleavage of the histidine tag from the A or A1 chain once it is no longer desired. Examples of protease-sensitive regions that may be used include a thrombin sensitive region or a furin sensitive region. Other protease-sensitive sequences are also well known in the art.

The invention will now be further described with reference to the following, nonlimiting examples.

### Example 1

### Proof-of-concept: design and mining of a prototypic A chain-tripeptide library

We originally created a simple tripeptide library inserted in the C-terminal protease-sensitive loop of the SLT-1 A chain (Figs. 2A and B). This A chain loop region is naturally constrained due to the presence of a single disulfide bond bridging Cys242 to Cys261. The maximal diversity of this library can thus be calculated to be 20³ or 8000 permutations of a tripeptide sequence. As a proof-of-concept that A chain libraries can easily be screened for a new receptor-binding activity, we picked more than 3000 colonies from this A chain-tripeptide library and purified the mutant toxin produced by each clone. We noticed very early in this study that the level of expression of A chain mutant was dramatically increased when expressed in the presence of the wild-type SLT-1 B subunit. Thus the mutant forms of A chain were expressed and initially purified as AB5 toxin variants. Since all A subunits harbor a polyHis purification tag, it is relatively easy to remove the B subunit with denaturants (urea for example) while recovering the A chain on metal-affinity columns or beads. Western blots performed on randomly selected bacterial clones indicated that > 70% of these colonies produced significant amounts of these A chain mutants.

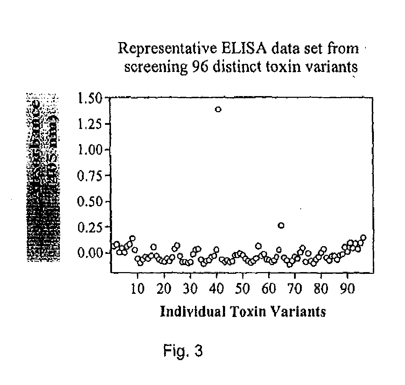

These toxin variants were then coated in individual wells of 96-well plates and screened by ELISA for their ability to bind the monoclonal antibody Onc M27 (Linsley, et al.(1988) Monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibody SM-3 is selectively exposed in a range of primary carcinomas. Int J Cancer 43, 1072-1076). As shown in Tables 1 and 2, most A chain mutants neither coded for the tripeptide insert that matched the targeted epitope of One M27 nor were recognized by the antibody. However on two occasions, a toxin variant harboring the exact epitope (Table 1, Fig.3) gave a strong ELISA signal comparable to the one observed with our control A chain harboring the MUC1 tripeptide epitope and had the expected epitope sequence in its randomized tripeptide region. A typical ELISA data set for 96 A chain mutants is presented in Fig. 3, highlighting the fact that the majority of A chain mutants did not recognized the mAb One M27 except for an A chain variant (mutant #41 in Tables 1 and 2). These results clearly established that A chain libraries can easily be constructed and screened to find toxin variants able to specifically target a given receptor, in this case an antigen-combining site.

### Example 2

### Proof-of-concept: design and mining of a prototypic A1 chain-tripeptide library

A tripeptide library inserted in the C-terminal SLT-1 A1 domain is prepared. A simple tripeptide library is inserted in the C-terminal protease-sensitive loop of the SLT-1 A chain (Figs. 2A and B). This A chain loop region is naturally constrained due to the presence of a single disulfide bond bridging Cys242 to Cys261. The maximal diversity of this library can thus be calculated to be 20³ or 8000 permutations of a tripeptide sequence. A chain libraries can easily be screened for a new receptor-binding activity. Over 3000 colonies from this A chain tripeptide library are picked and the mutant toxin produced by each clone is purified. The level of expression of A chain mutant is dramatically increased when expressed in the presence of the wild-type SLT-1 B subunit. Thus the mutant forms of A chain are expressed and initially purified as AB5 toxin variants. Since all A subunits harbor a polyHis purification tag, it is relatively easy to remove the B subunit with denaturants (urea for example) while recovering the A chain on metal-affinity columns or beads. Western blots performed on randomly selected bacterial clones indicate that > 70% of these colonies produce significant amounts of these A chain mutants. The A 1 subunit is then excised from the A chain mutants, using a protease cleavage using furin (Fig. 2G and H).

These toxin variants are then coated in individual wells of 96-well plates and screened by ELISA for their ability to bind the monoclonal antibody One M27 (Linsley, et al.(1988) monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibody SM-3 is selectively exposed in a range of primary carcinomas. Int J Cancer 43, 1072-1076). Most A1 chain mutants neither code for the tripeptide insert that match the targeted epitope of One M27 nor are recognized by the antibody. However on a few occasions, a toxin variant harboring the exact epitope gives a strong ELISA signal comparable to the one observed with our control A chain harboring the MUC1 tripeptide epitope and has the expected epitope sequence in its randomized tripeptide region. These results clearly establish that A1 chain libraries can easily be constructed and screened to find toxin variants able to specifically target a given receptor, in this case an antigen-combining site.

### Example 3

### Proof-of-concept: Alternative method for design and mining of a prototypic A1 chain-tripeptide library

A tripeptide library inserted in the C-terminal SLT-1 A1 domain is prepared. A simple tripeptide library is inserted in the C-terminal protease-sensitive loop of the SLT-1 A1 chain (Fig. 2 G and H). This A1 domain codes for A region amino acids 1-251. The maximal diversity of this library can thus be calculated to be 20³ or 8000 permutations of a tripeptide sequence. A chain libraries can easily be screened for a new receptor-binding activity. Over 3000 colonies from this A1 chain tripeptide library are picked and the mutant toxin produced by each clone is purified. Since all A1 subunits lack a B subunit binding area, it is relatively easy to exise any B subunits. Optionally, the polyHIS tags can be used to further purify the A1 subunits, as described in previous examples.

These toxin variants are then coated in individual wells of 96-well plates and screened by ELISA for their ability to bind the monoclonal antibody One M27 (Linsley, et al.(1988) monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibody SM-3 is selectively exposed in a range of primary carcinomas. Int J Cancer 43, 1072-1076). Most A1 chain mutants neither code for the tripeptide insert that match the targeted epitope of One M27 nor are recognized by the antibody. However on a few occasions, a toxin variant harboring the exact epitope gives a strong ELISA signal comparable to the one observed with our control A chain harboring the MUC1 tripeptide epitope and has the expected epitope sequence in its randomized tripeptide region. These results clearly establish that A1 chain libraries can easily be constructed and screened to find toxin variants able to specifically target a given receptor, in this case an antigen-combining site.

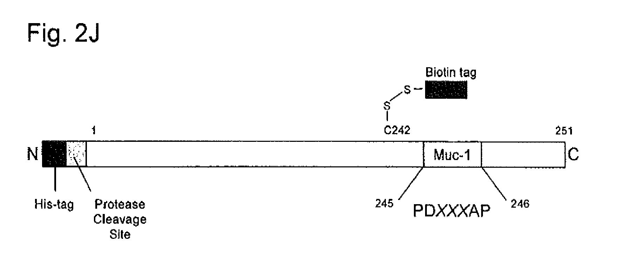

The A1 tripeptide library can also be biotinylated at the C242 position (Figure 2J). A standard biotin assay can then be used to determine whether an A1 tripeptide library member is present, or bound to a substrate or given receptor.

### Example 4

### Proof-of-concept: Method for design and mining of a prototypic A chain-tripeptide library with a mutation in C242 and/or C 261

A tripeptide library inserted in the C-terminal SLT-1 A domain is prepared, using the method of Example 1 with the modification that encoding for the cysteine at position 242 and/or position 261 has been mutated to an alanine using site directed mutagenesis (Figures 2C, 2D, or 2E). For libraries containing one cysteine in the A chain, the tripeptide library is optionally biotinylated (Fig. 2F). The A chain libraries can easily be screened for a new receptor-binding activity. Results indicate that these cys to ala modified A chain libraries are at least as effective as the wild-type libraries of Example 1 in ease of construction and screening to find toxin variants able to specifically target a given receptor. In the case of the biotinylated libraries, the biotin labels are easily used, utilizing known methods, to identify whether a member of the A chain library is present or bound to a sample.

### Example 5

### Proof-of-concept: Method for design and mining of a prototypic A1 chain-tripeptide library with a mutation in C242 and/or C 261

A tripeptide library inserted in the C-terminal SLT-1 A1 domain is prepared, using the method of Example 2 with the modification that encoding for the cysteine at position 242 and/or position 261 has been mutated to an alanine using site directed mutagenesis (Fig. 2I), The A1 chain libraries can easily be screened for a new receptor-binding activity. Results indicate that these cys to ala modified A1 chain libraries are as effective as the wild-type libraries of Example 2 in ease of construction and screening to find toxin variants able to specifically target a given receptor. Results indicate that these cys to ala modified A1 chain libraries are significantly easier to make and purify, due to the lack of the disulfide bridge between Cys 242 and Cys 261, resulting in a much easier cleavage of A2 from A1 in the protease cleavage step. In the case of an A1 tripeptide library having a cysteine at position C242, the cysteine is optionally tagged with biotin (Fig. 2J). This allows for determination of whether the library member is present, or bound to a substrate or receptor, using known methods.

### Example 6

### Proof-of-concept: Method for design and mining of a prototypic A1 chain-tripeptide library with a mutation in C242 and/or C 261

A tripeptide library inserted in the C-terminal SLT-1 A1 domain is prepared, using the method of Example 3 with the modification that encoding for the cysteine at position 242 has been mutated to an alanine using site directed mutagenesis (Fig. 2I). The A1 chain libraries can easily be screened for a new receptor-binding activity. Results indicate that these cys to ala modified A1 chain libraries are as effective as the wild-type libraries of Example 4 in ease of construction and screening to find variants able to specifically target a given receptor.

### Example 7

### Making of Combinatorial SLT-1 A-heptapeptide or A1-heptapeptide Library

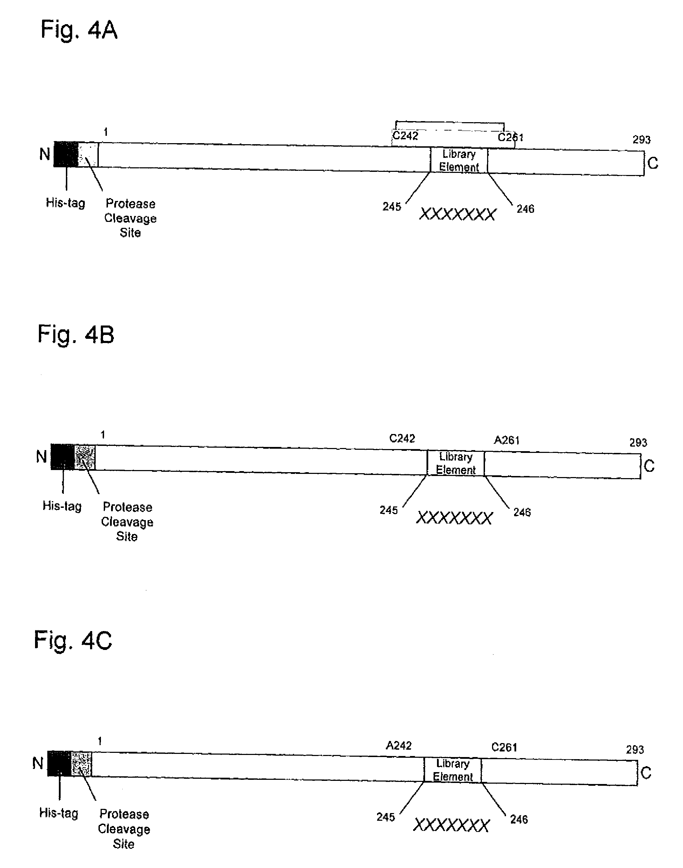

Library diversity represents a crucial parameter in screening combinatorial libraries for ligands able to bind specifically and with high affinity to a particular target. The SLT-1 A chain-tripeptide library described in Examples 1 or 4, and the SLT-1 A1 chain-tripeptide libraries described in Examples 2, 3, 5 and 6 have a maximal diversity of 20³ or 8000 possible mutated A chains. This sampling repertoire is small but was useful in mapping a tripeptide epitope to establish our proof-of-concept. A seven-residue library (20⁷ or 1.3 x 10⁹ possible mutants) represents a more typical minimal diversity level commonly used in designing phage display as well as synthetic peptide libraries. Thus, as a starting point, we build SLT-1 A and A1 chain libraries (as described in Examples 1-6) with a 7-amino acid long random sequence inserted in its C-terminus (Fig. 4A-H). Each of these libraries provide sufficient diversity to insure that A chain or A1 chain toxin variants can be identified that target new or known internalized receptors on cancer cells. All elements of each of these libraries (as well as all other libraries proposed) contain a N-terminal His tag to quickly purify A chain mutants. The libraries (Fig. 4A-4H) are generated using a megaprimer PCR strategy (Sarkar G, and Sommers S, (1990). The 'megaprimer' method of site-directed mutagenesis. Biotechniques 8, 404-407). The megaprimer strategy is widely used to introduce mutations into target DNA sequence, by way of two rounds of PCR that use two flanking primers and an internal mutagenic primer.

All A chain-heptapeptide libraries and A1 chain-hoptapeptide libraries are made, as follows. The SLT-1 A chain-heptapeptide libraries (Fig. 4A-4H) harbor a 7-amino acid random insertion between amino acid 245 and 246 of the A or A1 chain, the identical site used to construct our SLT-1 A chain-tripeptide library (Tables 1, 2, Fig. 3). Briefly, construction of the A chain heptapeptide libraries is described as follows using as the example an A chain containing 2 cysteines. Construction of the A1 chain heptapeptide libraries is undertaken using similar methods, with modifications as necessary to form an A1 chain (and as described in parallel Examples 2, 3, 5 and 6, above).

Two flanking primers A (GTT ACT GTG ACA GCT GAA GCT TTA CGT TTT CG (Seq. ID No. 2) and B (GAG AAG AAG AGA CTG CAG ATT CCA TCT GTT G (Seq. ID No. 3)) carrying Hind111 and Pst1 restriction sites respectively were annealed within the 5' and 3' ends of the SLT-1 operon. A library oligonucleotide F containing all seven random amino acid (NNS) as well as a long matching sequence to anneal to the template were synthesized. In the synthesis of the random oligonucleotide, the relative representation of each amino acid was improved by restricting the third position of each codon to G or T (Noren, K. A., and Noren, C. J. (2001) Construction of high-complexity combinatorial phage display peptide libraries. Methods 23, 169-178.). This type of restriction reduces the overall DNA sequence complexity as well as coding discrepancy between residues (Reidhaar-Olson, et al. (1991) Random mutagenesis of protein sequences using oligonucleotide cassettes. Methods Enzymol 208, 564-586.). This strategy also minimizes the occurrence of stop codons (TAA and TGA) while the stop codon (TAG) is suppressed by using a supE bacterial strain, which specifies for the insertion of a Gln residue when TAG codons are translated. The first PCR reaction was performed using primers A and F and the resulting product purified by denaturing polyacrylamide gel electrophoresis. This product then served as the megaprimer with primer B for a second PCR reaction to amplify the random DNA. The final library DNA (PCR product) will then be digested with Hind111 and Pst1 and cloned into the backbone of a pECHE3a expression vector (MTI, Toronto). The E. coli strain JM101 was subsequently transformer with the resulting pECHE vector and single bacterial colonies picked, lysed and their supernatants analyzed for the expression of single A chain toxins or layered on cancer cells and screened using the SRB for cell cytotoxicity assay.

### Example 8

### Mining the combinatorial SLT-1 A-heptapeptide library against cancer cell lines using a cytotoxicity assay

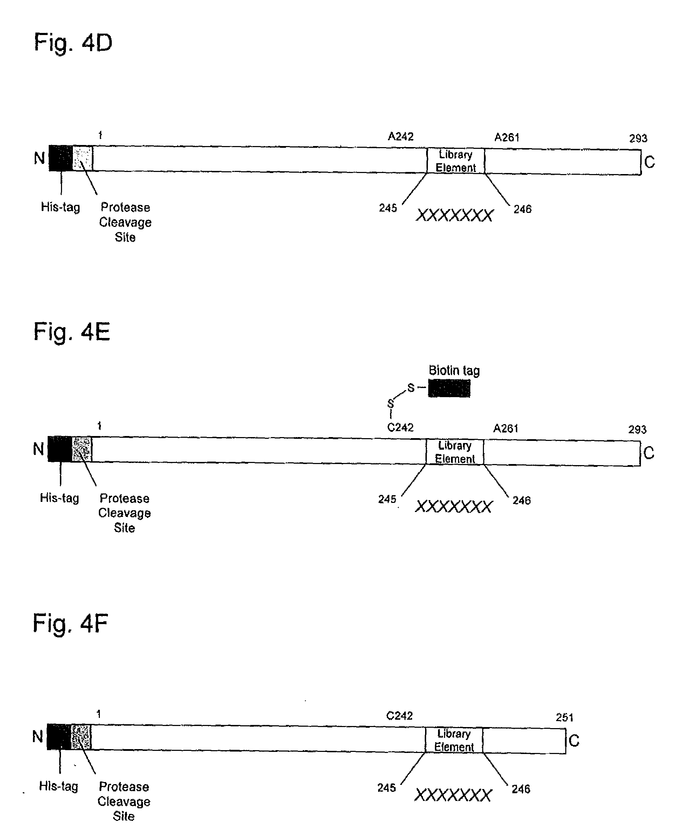

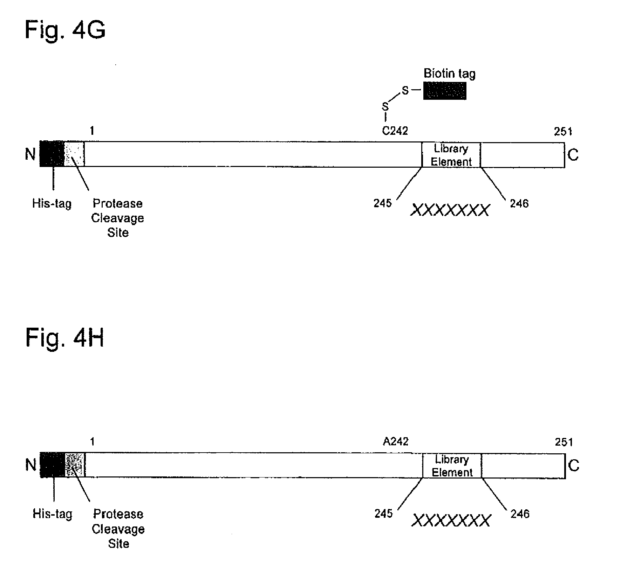

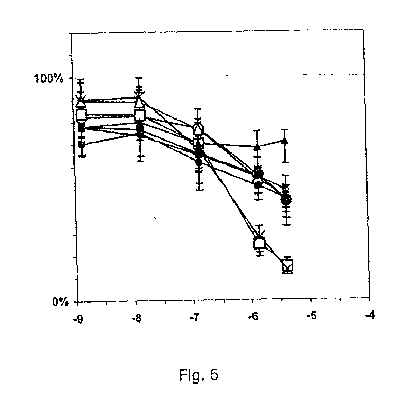

We screen our SLT-1 A-heptapeptide libraries and our SLT-1 A1-heptapeptide libraries using the cytotoxic function of the A or A1 chain as a reporter signal. Cytotoxicity is a more informative property to measure than binding to a receptor since it implies that the toxin is internalized, processed and delivered near ribosomes, clearly a multi-step event. The cytotoxicity assay is essentially performed as previously described (Bray, et al. (2001) Probing the surface of eukaryotic cells using combinatorial toxin libraries. Current Biology 11, 697-701). Briefly, the strategy to screen all our A chain libraries is based on the following principles. Established cancer cell lines such as SK-BR-3 (human breast), CAMA-1 (human breast), 518A2 (human melanoma), PC3 (human prostate) and B16 (murine melanoma) are grown in 96-well plates and used as targets in the primary screen stages. These cell lines are initially selected for our holotoxin library searches (Bray, supra) based on their adherence (plastic), their cell viability staining properties (SRB) in a high-throughput screening setting as well as their lack of receptor and sensitivity to native SLT-1 (to insure a reduced level of false positives). Single bacterial colonies from each library are picked and grown in 96 deep well plates. The cells are harvested, lysed, and their lysates clarified. Since all expressed SLT-1 A chain variants or A1 chain variants have a histidine tag at their N-terminus, each of them is purified from their lysate using nickel-affinity beads (96-well format) and layered on target cells. The plates containing the target cells treated with A chain variants or A1 chain variants are then incubated at 37°C for 48 hours, followed by fixation and staining with Sulforhodamine B (SRB). The SRB assay is a colorimetric end-point assay, which quantifies viable cells by staining their cellular protein content (Skehan, et al, (1990) New colorimetric cytotoxicity assay for anticancer-drug screening. J Natl Cancer Inst 82, 1107-1112.). The SRB assay has been adopted by NCI/NIH for their high-throughput screening of drug candidates on cancer cell lines. Viability assays are repeated for any bacterial extracts leading to cell death. A first round of screening is performed on more than 5000 bacterial clones (equivalent to 5000 distinct A chain toxins) for each of the A chain and A1 chain pentapeptide libraries as described in Figures 4A to 4H, and several toxin variants are identified in each library as repeatable killers of the human melanoma cell line 518A2 (Fig. 5). We find that the A1 chain libraries of figures 4 F, 4 G, and 4 H have similar cytotoxic properties as of that of the A chain libraries. We also find that the libraries with mutations at cysteine 242 or cysteine 261, or at both cysteines, as described in Figures 4B, 4C, 4D, and 4E also have similar cytotoxic properties as that of the "wild type" A chain library of Figure 4A.

The abscissa represents the log concentration of toxin used to treat the cells and the ordinate depicts the observed percentage of cells that are viable after 48 hours. The closed triangles depicts the effect of the wild type toxin on 518A2 cells while the two most efficacious A chain variants were termed SAM#3 (open squares) and SAM#5 (X symbols).

Promising A chain and A1 chain variants from each of the heptapeptide libraries are then re-screened against a panel of cell lines (Vero [Monkey, normal kidney]; PC-3 [Human, prostate cancer]; HepG2 [Human, hepatoma]; SiHa [Human, cervical cancer]; PanC [Human, pancreatic cancer]; SKBR-3 [Human, breast cancer]; 518-A2 [Human, melanoma]; U87 [Human, glioma]; B16-F10 [Mouse, melanoma]; HS-216 [Human, normal fibroblast]; CAMA-1 [Human, breast cancer]; OVCar-3 [Human, ovarian cancer]). Of these variants, a significant proportion are observed to have activity against each of the cancer cell line, the 518-A2 human melanoma, and the SiHa (human cervical cancer cells) and U87-A (human brain cancer cells; glioma) cell lines.

The genes coding for the two A chain toxins (SAM3 and SAM5) that resulted in toxicity of the human melanoma cell lines were sequenced to determine the amino acid sequences inserted between residues 245 and 246 of the wild-type A chain. The sequences, including the His-tag, are listed in Seq. ID Nos 4 and 5, respectively.

**Table 1: DNA sequences of randomly picked clones from the SLT-1 A chain-tripeptide library. Mutated bases in bold characters. Mutant #41 was identified in our ELISA screen as a strong binder of mAb Onc M27 (Fig. 3).**

| SLT-1 A chain Nucleotide sequence variant | | Diversity (nucleotide changes in mutated region) |
|---|---|---|
| MUC1 epitope | CCA GAC ACG CGA CCA GCT CCA | 0/9 |
| Mutant #1 | CCA GAC GGG ATC GGG GCT CCA | 8/9 |
| Mutant #2 | CCA GAC CTG GAG ATG GCT CCA | 8/9 |
| Mutant #3 | CCA GAC CCC CGT GGG GCT CCA | 6/9 |
| Mutant #4 | CCA GAC GAC GAC TTG GCT CCA | 9/9 |
| Mutant #5 | CCA GAC GTC CGG TGG GCT CCA | 7/9 |
| Mutant #6 | CCA GAC CAG CGC TGG GCT CCA | 6/9 |
| Mutant #7 | CCA GAC CTC AGG ATG GCT CCA | 8/9 |
| Mutant #8 | CCA GAC TCC CAG GAG GCT CCA | 7/9 |
| Mutant #9 | CCA GAC TCC GAC CCC GCT CCA | 6/9 |
| Mutant #41 | CCA GAC ACG CGC CCC GCT CCA | 2/9 |

**Table 2: Amino acid sequence alignment of randomly selected clones from the SLT-1 A chain-tripeptide library and ELISA signal of purified SLT-1 A chain variants detected with a mAb (Onc M27) raised against the MUC1 epitope Thr-Arg-Pro. Mutated tripeptide region in bold characters. Mutant #41 was identified in our ELISA screen as a strong binder of mAb Onc M27.**

| SLT-1 A chainDeduced amino acid sequence variant | | ELISA readings (405 nm) |
|---|---|---|
| MUC1 epitope | CHHHPD TRP APASRVARMASDEFPSMC | 1.3 |
| Mutant #1 | CHHHPD GIG APASRVARMASDEFPSMC | 0.08 |
| Mutant #2 | CHHHPD LQM APASRVARMASDEFPSMC | 0.03 |
| Mutant #3 | CHHHPD PRG APASRVARMASDEFPSMC | 0.03 |
| Mutant #4 | CHHHPD DDL APASRVARMASDEFPSMC | 0.06 |
| Mutant #5 | CHHHPD VRW APASRVARMASDEFPSMC | 0.07 |
| Mutant #6 | CHHHPD QRL APASRVARMASDEFPSMC | 0.06 |
| Mutant #7 | CHHHPD LRM APASRVARMASDEFPSMC | 0.11 |
| Mutant #8 | CHHHPD SQE APASRVARMASDEFPSMC | 0.13 |
| Mutant #9 | CHHHPD SDP APASRVARMASDEFPSMC | 0.07 |
| Mutant #41 | CHHHPD TRP APASRVARMASDEFPSMC | 1.25 |

### FURTHER EMBODIMENTS OF THE INVENTION

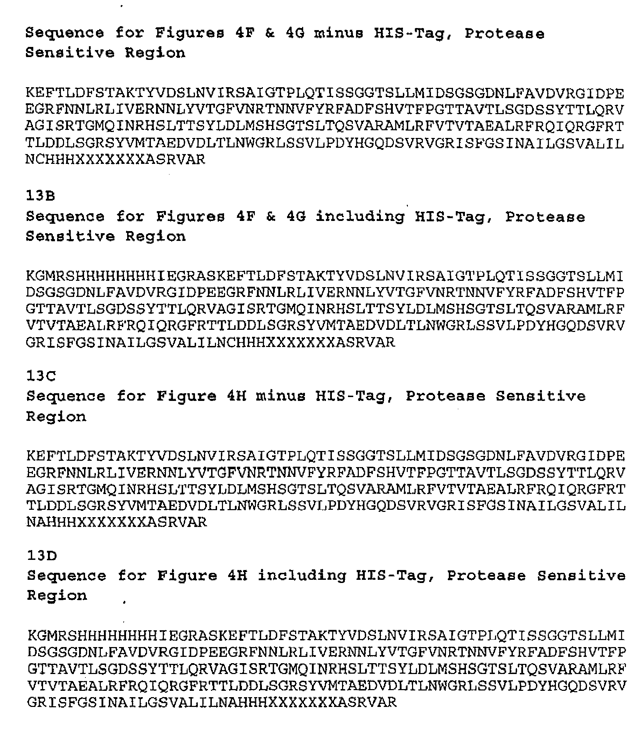

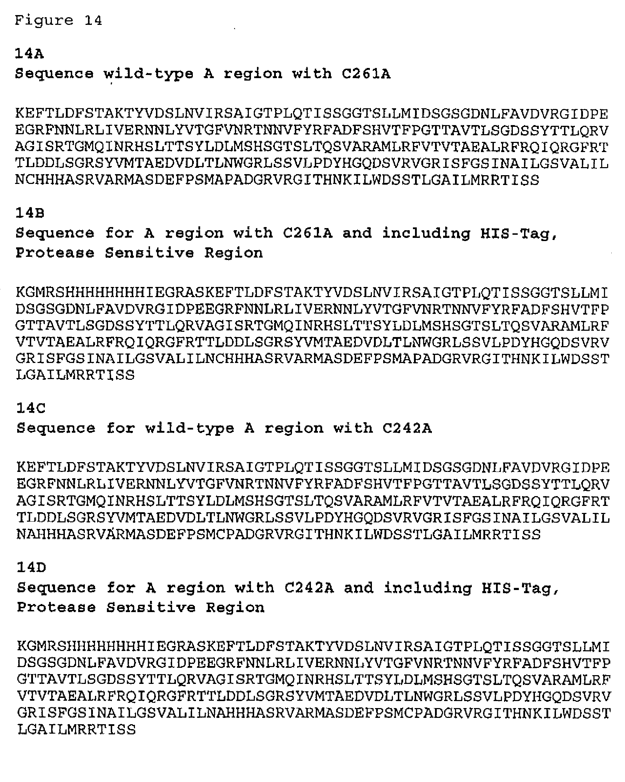

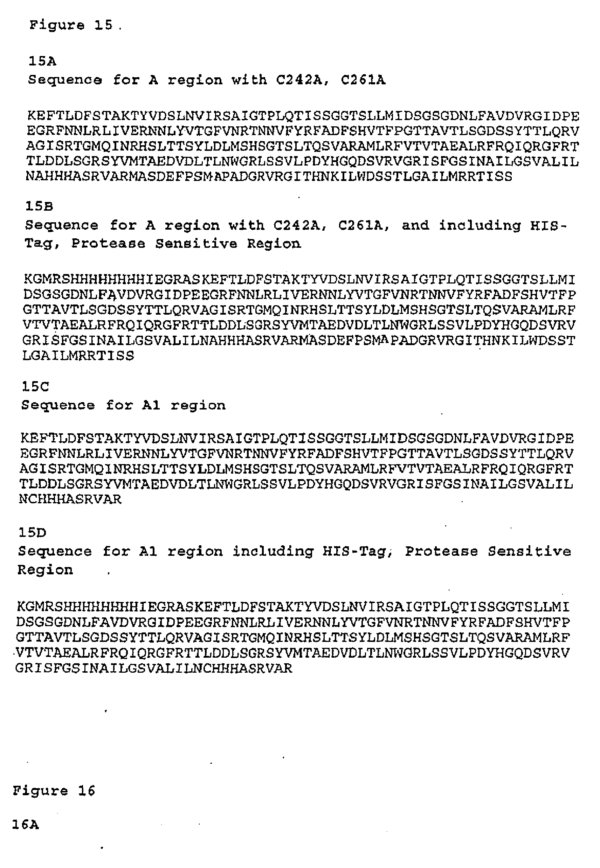

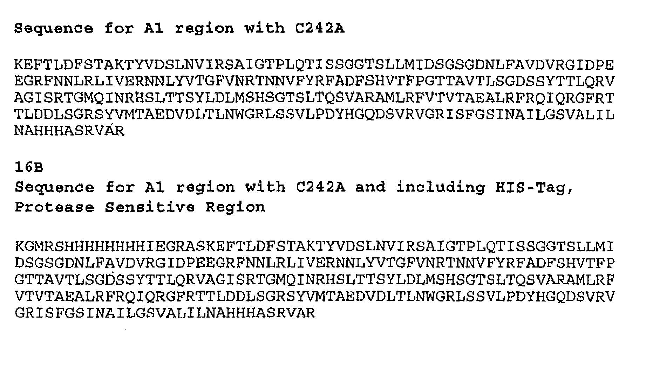

The invention also relates to the following Embodiments 1-128:
Embodiment 1. A combinatorial protein library comprising a plurality of protein species,
each protein species comprising an A chain of a toxic protein into which an insert has been introduced, wherein,
   (a) the insert is a polypeptide of varying amino acid sequence having a length of at least 2 amino acid residues; and
   (b) the insert is introduced into the protease-sensitive loop of the A chain sequence.
Embodiment 2. The combinatorial protein library of Embodiment 1, wherein the library comprises at least 100 protein species.
Embodiment 3. The combinatorial protein library of Embodiment 1 or 2, wherein the protein species are formed by introducing the insert into a Shiga-like toxin I A chain.
Embodiment 4. The combinatorial protein library of Embodiment 3, wherein the protein species is formed by introducing the insert between amino acids 242 and 261, as defined with reference to the sequence listed in Figure 6.
Embodiment 5. The combinatorial protein library of Embodiment 4, wherein the protein species is formed by introducing the insert between amino acids 245 and 246, as defined with reference to the sequence listed in Figure 6.
Embodiment 6. The combinatorial protein library of Embodiment 3, wherein the protein species is formed by introducing the insert before or after amino acids 1-239 of the Shiga-like toxin I A chain, as defined with reference to the sequence listed in Figure 6.
Embodiment 7. The combinatorial protein library of any preceding Embodiment, wherein the insert has a length of 7 amino acids.
Embodiment 8. A combinatorial expression library comprising a plurality of species of expression systems, each species expressing a protein species in accordance with any of Embodiment s 1-7.
Embodiment 9. A mutant protein comprising an A chain of a toxic protein into which an insert has been introduced, wherein,
   (a) the insert is a polypeptide of varying amino acid sequence having a length of at least 2 amino acid residues; and
   (b) the insert is introduced into the protease-sensitive loop of the A chain sequence,
Embodiment 10. The mutant protein of Embodiment 9, wherein the A chain of a toxic protein is a Shiga-like toxin I A chain.
Embodiment 11. The mutant protein of Embodiment 10, wherein the insert in introduced between amino acids 242 and 261, as defined with reference to the sequence listed in Figure 6.
Embodiment 12. The mutant protein of Embodiment 11, wherein the insert is introduced between amino acids 245 and 246, as defined with reference to the sequence listed in Figure 6.
Embodiment 13. The mutant protein of Embodiment 12, wherein the insert comprises the sequence IYSNKLM (Seq. ID No. 6).
Embodiment 14. The mutant protein of Embodiment 12, wherein the insert comprises the sequence AAFADLI (Seq. ID No. 7).
Embodiment 15. The mutant protein of Embodiment 10, wherein the insert is introduced before or after amino acids 1-239 of the Shiga-like toxin I A chain, as defined with reference to the sequence listed in Figure 6.
Embodiment 16. The mutant protein of any of Embodiments 9 to 15, wherein the insert has a length of 7 amino acids.
Embodiment 17. A method for identifying a ligand that bind to a specific target/receptor, comprising the steps of:
   (a) exposing cells known to possess the target/receptor to members of a combinatorial protein library in accordance with any of Embodiments I to 7;
   (b) selecting members of the protein library which are observed to be toxic to the cells;
   (c) evaluating the selected members of the protein library to determine the sequence of the inserted region, whereby a peptide of the sequence of the inserted region is identified as a possible ligand for a target/receptor on the cell; and
   (d) further testing peptides of the sequence of the inserted region to confirm that they are a ligand for the specific target/receptor.
Embodiment 18. A method for isolating a toxin specific for a known target/receptor comprising the steps of:
   (a) exposing the target/receptor to a combinatorial protein library according to any of Embodiments 1 to 7; and
   (b) isolating at least one protein species from the combinatorial protein library captured by binding to the target/receptor.
Embodiment 19. The method of Embodiment 18, further comprising the step of screening the isolated protein against cells expressing the target/receptor, to confirm their toxicity for cells expressing the target/receptor.
Embodiment 20. The method of Embodiment 18, wherein the target/receptor is a purified target/receptor and is immobilized on a solid support.
Embodiment 21. The method of Embodiment 20, wherein the target/receptor is on the surface of cells.
Embodiment 22. The method of Embodiment 21, wherein the cells are immobilized on a solid support.
Embodiment 23. The method of Embodiment 21 or 22, wherein the toxin serves as a reporter, and the death of the cells is indicative of receptor binding.
Embodiment 24. A combinatorial protein library comprising a plurality of protein species, each protein species comprising a modified A chain of a toxic protein into which an insert has been introduced, wherein,
   (a) the modified A chain comprises a wild-type A chain containing a mutation in at least one cysteine;
   (b) the insert is a polypeptide of varying amino acid sequence having a length of at least 2 amino acid residues; and
   (c) the insert is introduced into the region which would be a protease-sensitive loop in the wild-type A chain sequence.
Embodiment 25. The combinatorial protein library of Embodiment 24, wherein the library comprises at least 100 protein species.
Embodiment 26. The combinatorial protein library of Embodiment 24, wherein the protein species are formed by introducing the insert into a Shiga-like toxin I A chain containing a mutation in at least one cysteine.
Embodiment 27. The combinatorial protein library of Embodiment 24, 25 or 26, wherein the mutation in at least one cysteine is a mutation from cysteine to an alanine.
Embodiment 28. The combinatorial protein library of Embodiment 26, wherein the protein species is formed by introducing the insert between amino acids 242 and 261, as defined with reference to the sequence listed in figures 14A, 14C, or 15A.
Embodiment 29. The combinatorial protein library of Embodiment 26, wherein the protein species is formed by introducing the insert between amino acids 245 and 246, as defined with reference to the sequence listed in figures 14A, 14C or 15A.
Embodiment 30. The combinatorial protein library of Embodiment 26, wherein the protein species is formed by introducing the insert before or after amino acids 1-239 of the Shiga-like toxin I A chain, as defined with reference to the sequence listed in figures 14A, 14C or 15A.
Embodiment 31. The combinatorial protein library of any of Embodiments 24-30, wherein the insert has a length of 7 amino acids.
Embodiment 32. A combinatorial expression library comprising a plurality of species of expression systems, each species expressing a protein species in accordance with any of Embodiments 24-31.
Embodiment 33. The combinatorial expression library of any one of Embodiments 24 to 32, wherein the modified A chain comprises one cysteine, further comprising a label bound to said cysteine.
Embodiment 34. The combinatorial expression library of Embodiment 33 wherein the label is a fluorescent label.
Embodiment 35. The combinatorial expression library of Embodiment 33 wherein the label is biotinylation or pegylation..
Embodiment 36. The combinatorial expression library of Embodiment 33 wherein the label is a radioactive label.
Embodiment 37. A mutant protein comprising a modified A chain of a toxic protein into which an insert has been introduced, wherein,
   (a) the modified A chain comprises a wild-type A chain containing a mutation in at least one cysteine;
   (b) the insert is a polypeptide of varying amino acid sequence having a length of at least 2 amino acid residues; and
   (b) the insert is introduced into a region which would be a protease-sensitive loop in the wild type A chain sequence.
Embodiment 38. The mutant protein of Embodiment 37, wherein the mutation in at least one cysteine is a mutation from cysteine to an alanine.
Embodiment 39. The mutant protein of Embodiment 37, wherein the modified A chain comprises one cysteine, further comprising a label bound to said cysteine.
Embodiment 40. The mutant protein of Embodiment 39 wherein the label is a fluorescent label.
Embodiment 41. The mutant protein of Embodiment 39 wherein the label is biotinylation or pegylation.
Embodiment 42. The mutant protein of Embodiment 39 wherein the label is a radioactive label.
Embodiment 43. The mutant protein of Embodiment 37, wherein the wild-type A chain of a toxic protein is a Shiga-like toxin I A chain.
Embodiment 44. The mutant protein of Embodiment 37, wherein the insert in introduced between amino acids 242 and 261, as defined with reference to the sequences listed in figures 14A, 14C or 15A.
Embodiment 45. The mutant protein of Embodiment 37, wherein the insert is introduced between amino acids 245 and 246, as defined with reference to the sequences listed in figures 14A, 14C or 15A.
Embodiment 46. The mutant protein of Embodiment 45, wherein the insert comprises the sequence IYSNKLM (Seq. ID No. 6).
Embodiment 47. The mutant protein of Embodiment 45, wherein the insert comprises the sequence AAFADLI (Seq. ID No. 7).
Embodiment 48. The mutant protein of Embodiment 37, wherein the insert is introduced before or after amino acids 1-239 of a modified Shiga-like toxin I A chain, as defined with reference to the sequences listed in figures 14A, 14C or 15A.
Embodiment 49. The mutant protein of any of Embodiments 37 to 48, wherein the insert has a length of 7 amino acids.
Embodiment 50. A polypeptide sequence selected from the group consisting of the polypeptide sequence listed in figures 11C, 11D, 12A, 12B, 12C, or 12D.
Embodiment 51. A method for identifying a ligand that bind to a specific target/receptor, comprising the steps of:
   (a) exposing cells known to possess the target/receptor to members of a combinatorial protein library in accordance with any of Embodiments 24 to 36;
   (b) selecting members of the protein library which are observed to be toxic to the cells;
   (c) evaluating the selected members of the protein library to determine the sequence of the inserted region, whereby a peptide of the sequence of the inserted region is identified as a possible ligand for a target/receptor on the cell; and
   (d) further testing peptides of the sequence of the inserted region to confirm that they are a ligand for the specific target/receptor.
Embodiment 52. A method for isolating a toxin specific for a known target/receptor comprising the steps of:
   (a) exposing the target/receptor to a combinatorial protein library according to any of Embodiments 24 to 36; and
   (b) isolating at least one protein species from the combinatorial protein library captured by binding to the target/receptor.
Embodiment 53. The method of Embodiment 52, further comprising the step of screening the isolated protein against cells expressing the target/receptor, to confirm their toxicity for cells expressing the target/receptor.
Embodiment 54. The method of Embodiment 52, wherein the target/receptor is a purified target/receptor and is immobilized on a solid support.
Embodiment 55. The method of Embodiment 54, wherein the target/receptor is on the surface of cells.
Embodiment 56. The method of Embodiment 55, wherein the cells are immobilized on a solid support.
Embodiment 57. The method of Embodiment 55 or 56, wherein the toxin serves as a reporter, and the death of the cells is indicative of receptor binding.
Embodiment 58. A combinatorial protein library comprising a plurality of protein species, each protein species comprising an A1 chain of a toxic protein into which an insert has been introduced, wherein,
   (a) the insert is a polypeptide of varying amino acid sequence having a length of at least 2 amino acid residues; and
   (b) the insert is introduced into the portion of the A1 chain that would be a protease-sensitive loop if the A1 chain was a full-length A chain.
Embodiment 59. The combinatorial protein library of Embodiment 58, wherein the library comprises at least 100 protein species.
Embodiment 60. The combinatorial protein library of Embodiment 58 or 59, wherein the protein species are formed by introducing the insert into a Shiga-like toxin I A 1 chain.
Embodiment 61. The combinatorial protein library of Embodiment 60, wherein the protein species is formed by introducing the insert between amino acids 242 and 251, as defined with reference to figure 15C.
Embodiment 62. The combinatorial protein library of Embodiment 61, wherein the protein species is formed by introducing the insert between amino acids 245 and 246, as defined with reference to figure 15C.
Embodiment 63. The combinatorial protein library of Embodiment 60, wherein the protein species is formed by introducing the insert before or after amino acids 1-239 of the Shiga-like toxin I A1 chain, as defined with reference to figure 15C.
Embodiment 64. The combinatorial protein library of any one of Embodiments 58-63, wherein the insert has a length of 7 amino acids,
Embodiment 65. A combinatorial expression library comprising a plurality of species of expression systems, each species expressing a protein species in accordance with any of Embodiments 58-64.
Embodiment 66. A combinatorial expression library of any one of Embodiments 58-65 further comprising a label bound to a cysteine in said A1 chain.
Embodiment 67. The combinatorial expression library of Embodiment 66 wherein the label is a fluorescent label.
Embodiment 68. The combinatorial expression library of Embodiment 66 wherein the label is biotinylation or pegylation.
Embodiment 69. The combinatorial expression library of Embodiment 66 wherein the label is a radioactive label.
Embodiment 70. A mutant protein comprising an A1 chain of a toxic protein into which an insert has been introduced, wherein,
   (a) the insert is a polypeptide of varying amino acid sequence having a length of at least 2 amino acid residues; and
   (b) the insert is introduced into the portion of the A1 chain that would be a protease-sensitive loop if the A1 chain was a full length A chain.
Embodiment 71. The mutant protein of Embodiment 70 further comprising a label bound to a cysteine in said A1 chain.
Embodiment 72. The mutant protein of Embodiment 71 wherein the label is a fluorescent label.
Embodiment 73. The mutant protein of Embodiment 71 wherein the label is biotinylation or pegylation.
Embodiment 74. The mutant protein of Embodiment 71 wherein the label is a radioactive label.
Embodiment 75. The mutant protein of Embodiment 70, wherein the A1 chain of a toxic protein is a Shiga-like toxin I A1 chain.
Embodiment 76. The mutant protein of Embodiment 75, wherein the insert in introduced between amino acids 242 and 251, as defined with reference to figure 15C.
Embodiment 77. The mutant protein of Embodiment 76, wherein the insert is introduced between amino acids 245 and 246, as defined with reference to figure 15C.
Embodiment 78. The mutant protein of Embodiment 77, wherein the insert comprises the sequence IYSNKLM (Seq. ID No. 6).
Embodiment 79. The mutant protein of Embodiment 77, wherein the insert comprises the sequence AAFADLI (Seq. ID No. 7).
Embodiment 80. The mutant protein of Embodiment 75, wherein the insert is introduced before or after amino acids 1-239 of the Shiga-like toxin I A1 chain, as defined with reference to figure 15C.
Embodiment 81. The mutant protein of any of Embodiments 70 to 80, wherein the insert has a length of 7 amino acids.
Embodiment 82. A method for identifying a ligand that bind to a specific target/receptor, comprising the steps of:
   (a) exposing cells known to possess the target/receptor to members of a combinatorial protein library in accordance with any of Embodiments 58 to 69;
   (b) selecting members of the protein library which are observed to be toxic to the cells;
   (c) evaluating the selected members of the protein library to determine the sequence of the inserted region, whereby a peptide of the sequence of the inserted region is identified as a possible ligand for a target/receptor on the cell; and
   (d) further testing peptides of the sequence of the inserted region to confirm that they are a ligand for the specific target/receptor.
Embodiment 83. A method for isolating a toxin specific for a known target/receptor comprising the steps of:
   (a) exposing the target/receptor to a combinatorial protein library according to any of Embodiments 58 to 69; and
   (b) isolating at least one protein species from the combinatorial protein library captured by binding to the target/receptor.
Embodiment 84. The method of Embodiment 83, further comprising the step of screening the isolated protein against cells expressing the target/receptor, to confirm their toxicity for cells expressing the target/receptor.
Embodiment 85. The method of Embodiment 83, wherein the target/receptor is a purified target/receptor and is immobilized on a solid support.
Embodiment 86. The method of Embodiment 85, wherein the target/receptor is on the surface of cells.
Embodiment 87. The method of Embodiment 86, wherein the cells are immobilized on a solid support.
Embodiment 88. The method of Embodiment 86 or 87, wherein the toxin serves as a reporter, and the death of the cells is indicative of receptor binding.
Embodiment 89. A combinatorial protein library comprising a plurality of protein species, each protein species comprising a modified A1 chain of a toxic protein into which an insert has been introduced, wherein,
   (a) the modified A1 chain comprises a wild-type A1 chain containing a mutation in one cysteine;
   (b) the insert is a polypeptide of varying amino acid sequence having a length of at least 2 amino acid residues; and
   (c) the insert is introduced into the portion of the A1 chain that would be a protease-sensitive loop if the A1 chain was a full length A chain.
Embodiment 90. The combinatorial protein library of Embodiment 89, wherein the library comprises at least 100 protein species.
Embodiment 91. The combinatorial protein library of Embodiment 89 or 90, wherein the protein species are formed by introducing the insert into a Shiga-like toxin I A1 chain containing a mutation in one cysteine.
Embodiment 92. The combinatorial protein library of any one of Embodiments 89 to 91, wherein the mutation in one cysteine is that the cysteine is modified to an alanine.
Embodiment 93. The combinatorial protein library of Embodiment 91, wherein the protein species is formed by introducing the insert between amino acids 242 and 261, as defined with reference to figure 16A.
Embodiment 94. The combinatorial protein library of Embodiment 93, wherein the protein species is formed by introducing the insert between amino acids 245 and 246, as defined with reference to figure 16A.
Embodiment 95. The combinatorial protein library of Embodiment 91, wherein the protein species is formed by introducing the insert before or after amino acids 1-239 of the Shiga-like toxin I A1 chain, as defined with reference to figure 16A.
Embodiment 96. The combinatorial protein library of any of Embodiments 89-95, wherein the insert has a length of 7 amino acids.
Embodiment 97. A combinatorial expression library comprising a plurality of species of expression systems, each species expressing a protein species in accordance with any of Embodiments 89-96.
Embodiment 98. A mutant protein comprising a modified A1 chain of a toxic protein into which an insert has been introduced, wherein,
   (a) the modified A1 chain comprises a wild-type A1 chain containing a mutation in one cysteine;
   (b) the insert is a polypeptide of varying amino acid sequence having a length of at least 2 amino acid residues; and
   (b) the insert is introduced into the portion of the A1 cahin that would be a protease-sensitive loop if the A1 chain was a full-length A chain.
Embodiment 99. The mutant protein of Embodiment 98, wherein the wild-type A 1 chain of a toxic protein is a Shiga-like toxin I A1 chain.
Embodiment 100. The mutant protein of Embodiment 99, wherein the insert in introduced between amino acids 242 and 261, as defined with reference to figure 16A.
Embodiment 101. The mutant protein of Embodiment 100, wherein the insert is introduced between amino acids 245 and 246, as defined with reference to figure 16A.
Embodiment 102. The mutant protein of Embodiment 101, wherein the insert comprises the sequence IYSNKLM (Seq. ID No. 6).
Embodiment 103. The mutant protein of Embodiment 101, wherein the insert comprises the sequence AAFADLI (Seq. ID No. 7).
Embodiment 104. The mutant protein of Embodiment 99, wherein the insert is introduced before or after amino acids 1-239 of a modified Shiga-like toxin I A1 chain, as defined with reference to figure 16A.
Embodiment 105. The mutant protein of any of Embodiments 98 to 104, wherein the insert has a length of 7 amino acids.
Embodiment 106. A polypeptide sequence selected from the group consisting of the polypeptide sequence of figures 13A, 13B, 13C, 13D, 16A, or 16B,.
Embodiment 107. A method for identifying a ligand that bind to a specific target/receptor, comprising the steps of:
   (a) exposing cells known to possess the target/receptor to members of a combinatorial protein library in accordance with any of Embodiments 89 to 97;
   (b) selecting members of the protein library which are observed to be toxic to the cells;
   (c) evaluating the selected members of the protein library to determine the sequence of the inserted region, whereby a peptide of the sequence of the inserted region is identified as a possible ligand for a target/receptor on the cell; and
   (d) further testing peptides of the sequence of the inserted region to confirm that they are a ligand for the specific target/receptor.
Embodiment 108. A method for isolating a toxin specific for a known target/receptor comprising the steps of:
   (a) exposing the target/receptor to a combinatorial protein library according to any of Embodiments 89 to 97; and
   (b) isolating at least one protein species from the combinatorial protein library captured by binding to the target/receptor.
Embodiment 109. The method of Embodiment 108, further comprising the step of screening the isolated protein against cells expressing the target/receptor, to confirm their toxicity for cells expressing the target/receptor.
Embodiment 110. The method of Embodiment 108, wherein the target/receptor is a purified target/receptor and is immobilized on a solid support.
Embodiment 111. The method of Embodiment 108, wherein the target/receptor is on the surface of cells.
Embodiment 112. The method of Embodiment 111, wherein the cells are immobilized on a solid support.
Embodiment 113. The method of Embodiment 111 or 112, wherein the toxin serves as a reporter, and the death of the cells is indicative of receptor binding.
Embodiment 114. The combinatorial protein library of any one of Embodiments 1-8 wherein the insert is introduced at the N-terminus of the A chain sequence instead of in the protease sensitive loop region.
Embodiment 115. The mutant protein of any one of Embodiments 9-16 wherein the insert is introduced at the N-terminus of the A chain sequence instead of in the protease sensitive loop region.
Embodiment 116. The combinatorial protein library of any one of Embodiments 24-36 wherein the insert is introduced at the N-termius of the modified A chain instead of in the region that would be a protease sensitive loop in the wild-type A chain sequence.
Embodiment 117. The mutant protein of any one of Embodiments 37-49 wherein the insert is introduced at the N-termius of the modified A chain instead of in the region that would be a protease sensitive loop in the wild-type A chain sequence.
Embodiment 118. The combinatorial protein library of any one of Embodiments 58-69 or 89-97 wherein the insert is introduced at the N-terminus of the A1 chain instead of into the portion of the A1 chain that would be a protease-sensitive loop if the A1 chain was a full length A chain.
Embodiment 119. The mutant protein of any one of Embodiments 70-81 or 98-105 wherein the insert is introduced at the N-terminus of the A1 chain instead of into the portion of the A1 chain that would be a protease-sensitive loop if the A1 chain was a full length A chain.
Embodiment 120. A method for identifying a ligand that bind to a specific target/receptor, comprising the steps of:
   (a) exposing cells known to possess the target/receptor to members of a combinatorial protein library in accordance with any of Embodiments 114, 116, or 118;
   (b) selecting members of the protein library which are observed to be toxic to the cells;
   (c) evaluating the selected members of the protein library to determine the sequence of the inserted region, whereby a peptide of the sequence of the inserted region is identified as a possible ligand for a target/receptor on the cell; and
   (d) further testing peptides of the sequence of the inserted region to confirm that they are a ligand for the specific target/receptor.
Embodiment 121. A method for isolating a toxin specific for a known target/receptor comprising the steps of:
   (a) exposing the target/receptor to a combinatorial protein library according to any of Embodiments 114, 116 or 118; and
   (b) isolating at least one protein species from the combinatorial protein library captured by binding to the target/receptor.
Embodiment 122. The method of Embodiment 121, further comprising the step of screening the isolated protein against cells expressing the target/receptor, to confirm their toxicity for cells expressing the target/receptor.
Embodiment 123. The method of Embodiment 121, wherein the target/receptor is a purified target/receptor and is immobilized on a solid support.
Embodiment 124. The method of Embodiment 121, wherein the target/receptor is on the surface of cells.
Embodiment 125. The method of Embodiment 124, wherein the cells are immobilized on a solid support.
Embodiment 126. The method of Embodiment 124 or 125, wherein the toxin serves as a reporter, and the death of the cells is indicative of receptor binding.
Embodiment 127. The method of any one of Embodiments I to 23 or 82 to 88, wherein the library is biotinylated.
Embodiment 128. The library of any one of Embodiments 24 to 36 or 89 to 97, wherein the library is biotinylated.

## Claims

1. A combinatorial protein library comprising a plurality of protein species, each protein species comprising an A chain of a toxic protein modified by a mutation in at least one cysteine, the modified A chain further comprising a protease-sensitive loop or region into which an insert has been introduced, the insert comprising a polypeptide of varying amino acid sequence, wherein cleavage in the protease-sensitive loop or region releases an A chain sequence which, if otherwise unmodified, retains A chain catalytic activity.

2. The combinatorial protein library of claim 1, wherein the library comprises at least 100 protein species.

3. A mutant protein comprising an A chain of a toxic protein modified by a mutation in at least one cysteine, the modified A chain further comprising a protease-sensitive loop or region into which an insert has been introduced, the insert comprising a polypeptide of varying amino acid sequence, wherein cleavage in the protease-sensitive loop or region releases an A chain sequence which, if otherwise unmodified, retains A chain catalytic activity.

4. The combinatorial protein library or the mutant protein of any one of the above claims, wherein the toxic protein is an ABx toxic protein.

5. The combinatorial protein library or the mutant protein of any one of the above claims, wherein the A chain is a Shiga-like toxin I A chain.

6. The combinatorial protein library or the mutant protein of any one of the above claims, wherein the A chain is an A1 chain, and wherein the insert has been introduced into the region of the A1 chain that would be a protease-sensitive loop or region if the A1 chain were a full-length A chain.

7. The combinatorial protein library or the mutant protein of any one of the above claims, wherein the protein species or the mutant protein is formed by introducing the insert between amino acids 242 and 261, as defined with reference to the sequence listed in Figures 14A, 14C, or 15A, or between amino acids 242 and 261, as defined with reference to the sequence listed in Figure 16A.

8. The combinatorial protein library or the mutant protein of any one of the above claims, wherein the protein species or the mutant protein is formed by introducing the insert between amino acids 245 and 246, as defined with reference to the sequence listed in figures 14A, 14C, 15A, or 16A.

9. The combinatorial protein library or the mutant protein of any one of the above claims, wherein the protein species or the mutant protein is formed by introducing the insert before or after amino acids 1-239 of the Shiga-like toxin I A chain, as defined with reference to the sequence listed in figures 14A, 14C, 15A, or 16A.

10. The combinatorial protein library or the mutant protein of any one of the above claims, wherein the insert comprises a length of 7 amino acids.

11. The mutant protein of any one of claims 3 to 10, wherein the insert comprises the sequence IYSNKLM (Seq. ID No. 6).

12. The mutant protein of any one of claims 3 to 10, wherein the insert comprises the sequence AAFADLI (Seq. ID No. 7).

13. The combinatorial protein library or the mutant protein of any one of the above claims, wherein the mutation in at least one cysteine is a mutation from cysteine to alanine.

14. The combinatorial protein library or the mutant protein of any one of the above claims, wherein the modified A chain comprises a cysteine, and further comprises a label or probe bound to the cysteine.

15. The combinatorial protein library or the mutant protein of claim 14, wherein the label or probe is selected from the group consisting of: spectroscopic, radioactive, biochemical, fluorescent, biotin, and PEG labels or probes.

16. The combinatorial protein library or the mutant protein of any one of the above claims, wherein the modified A chain comprises an A chain sequence having catalytic activity.

17. The combinatorial protein library or the mutant protein of any one of the above claims, wherein the modified A chain comprises at least one additional mutation which reduces or abolishes A chain catalytic activity.

18. The combinatorial protein library or the mutant protein of any one of the above claims, wherein the insert is introduced at the N-terminus of the modified A chain instead of in the region that would be a protease-sensitive loop or region in the wild-type full-length A chain sequence.

19. A combinatorial expression library comprising a plurality of species of expression systems, each species expressing a mutant protein or protein species in accordance with any one of claims 1-18.

20. A polypeptide sequence selected from the group consisting of the polypeptide sequence listed in Figures 11C, 11D, 12A, 12B, 12C, 12D, 13C, 13D, 16A, or 16B.

21. A method for identifying a ligand that binds to a specific target/receptor, comprising the steps of:
(a) exposing cells known to possess the target/receptor to members of a combinatorial protein library in accordance with any of claims 1 to 2, 4 to 10, or 13 to 18; and
(b) selecting a member of the protein library which are observed to be toxic to the cells.

22. The method of claim 21, further comprising the step of evaluating the selected member of the protein library to determine the sequence of the inserted region, whereby a peptide of the sequence of the inserted region is identified as a possible ligand for a target/receptor on the cell.

23. The method of any one of claims 21 to 22, further comprising testing a selected member of the protein library to confirm that it is a ligand for the specific target/receptor.

24. A method for isolating a toxin specific for a target/receptor comprising the steps of:
(a) exposing the target/receptor to a combinatorial protein library according to any of claims 1 to 2, 4 to 10, or 13 to 18; and
(b) isolating at least one protein species from the combinatorial protein library captured by binding to the target/receptor.

25. The method of claim 24, wherein the target/receptor is a purified target/receptor and is immobilized on a solid support.

26. The method of any one of claims 24 to 25, further comprising the step of screening the isolated protein against cells expressing the target/receptor, to confirm its toxicity for cells expressing the target/receptor.

27. The method of claim 26, wherein the target/receptor is on the surface of cells.

28. The method of any one of claims 26 to 27, wherein the cells are immobilized on a solid support.

29. The method of any one of claims 26 to 28, wherein the toxin serves as a reporter, and the death of the cells is indicative of receptor binding.

30. A method for identifying a toxin for a cell expressing a specific target/receptor, comprising the steps of:
(a) exposing cells known to possess the target/receptor to members of a combinatorial protein library according to any of claims 1 to 2, 4 to 10, or 13 to 18; and
(b) selecting members of the protein library that are observed to be toxic to the cells, wherein members of the protein library that are observed to be toxic to the cells are toxins for the cells expressing the specific target/receptor.

31. The method of claim 30, further comprising the step of identifying a ligand that binds to a specific target/receptor, comprising the steps of:
(c) exposing cells known to possess the target/receptor to members of a combinatorial protein library according to any of claims 1 to 2, 4 to 10, or 13 to 18; and
(d) selecting members of the protein library that are observed to be toxic to the cells, wherein members of the protein library that are observed to be toxic to the cells are ligands that bind to the specific target/receptor.

32. The method of any one of claims 30 to 31, further comprising a step or steps of mutating an insert to further increase the toxicity of the protein species as compared to the protein species comprising the original insert.

33. A method of increasing the toxicity of the mutant protein of any one of claims 3 to 18, comprising the steps of:
(a) mutating a known insert possessing toxicity to cells expressing a specific target/receptor; and
(b) comparing toxicity of the protein species comprising the mutated insert to the protein species comprising the original insert.

34. The method of any one of claims 30 to 33, further comprising a step of evaluating the selected members of the protein library to determine the sequence of the inserted region.

35. The method of any one of claims 30 to 34, further comprising a step of further testing the selected members of the protein library to confirm that they are toxic to a cell expressing a specific target/receptor.

36. The method of any one of claims 21 to 23 or 26 to 35, wherein the cells are cancer cells.

37. The method of claim 36, wherein the cancer cells are selected from the group consisting of melanoma cells, cervical cancer cells, or brain cancer cells.
